# EUROPEAN PATENT APPLICATION

(11) **EP 4 825 861 A1**
(43) Date of publication of application: **21.10.2026**
(21) Application number: 23961239.3
(22) Date of filing: 19.12.2023
(51) Int. Cl.: C07F 5/02, C07D 471/22, C09K 11/06, H10K 50/11

(54) **ORGANIC COMPOUND AND DISPLAY PANEL**

(30) Priority: 13.12.2023 CN 202311715521
(71) Applicant: Wuhan China Star Optoelectronics Semiconductor Display Technology Co., Ltd., Wuhan, Hubei 430079 (CN)
(72) Inventor: NING, Weimin, Wuhan, Hubei 430079 (CN)
(74) Representative: Nederlandsch Octrooibureau
(86) International application number: PCT/CN2023/139693
(87) International publication number: WO 2025/123383

(57) **Abstract**

Embodiments of the present application provide an organic compound and a display panel. The organic compound has a structure represented by formula (1):

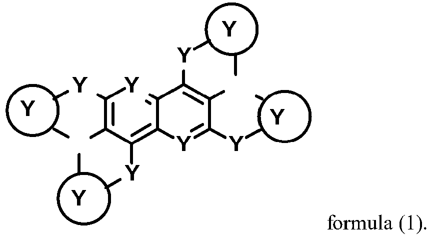

## Description

### TECHNICAL FIELD

The present application relates to the field of display, and in particular, to an organic compound and a display panel.

### BACKGROUND

An organic electroluminescent element generally has a positive electrode, a negative electrode, and an organic layer disposed between the positive electrode and the negative electrode. The organic substance in the organic layer is used to convert electrical energy into light energy, thereby realizing organic electroluminescence. A voltage is applied between the positive electrode and the negative electrode of the organic electroluminescent element, holes in the positive electrode are injected into the organic layer, and electrons in the negative electrode are injected into the organic layer, then the injected holes meet with the injected electrons to form excitons, which emit light when they transition back to a ground state, thus realizing luminescence of the organic electroluminescent element. The organic electroluminescent element has broad application prospect due to its characteristics such as autonomous luminescence, high brightness, high efficiency, low driving voltage, wide viewing angle, high contrast, high responsiveness, and the like. With the increasing needs for display effect in recent years, the development of high color gamut display devices has become a focus of attention for users. Developing materials that can realize higher luminescence efficiency and higher color purity of the organic electroluminescent element to meet the needs for high color gamut display devices is a key direction of attention. At present, in order to improve color purity, life, and luminescence efficiency of the organic electroluminescent element to obtain display panels with better display performances, materials used in the organic layer (such as the light-emitting layer) of the organic electroluminescent element still need to be improved.

Therefore, an organic compound and a display panel are needed to solve the technical problems.

### TECHNICAL PROBLEM

The present application provides an organic compound and a display panel. By providing the organic compound with a multi-resonance effect and elongating the distance between B and B atoms in the organic compound and/or introducing N atom(s) to break conjugation to weaken para-electronic coupling, the present application improves properties of the organic compound, thereby improving luminescence efficiency and color purity of the display panel using the organic compound, and prolonging service life of the display panel.

### TECHNICAL SOLUTIONS

To solve the problems mentioned above, the technical solutions provided by the present application are as follows.

The present application provides an organic compound having a structure represented by formula (1): 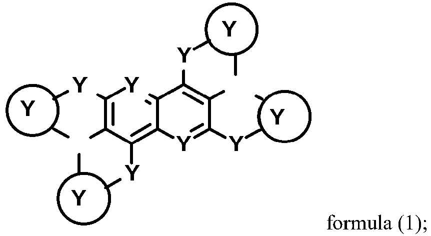
in which M₁ and M₂ are each independently selected from B or N;
Y₁ and Y₂ are each independently selected from C or N, and at least one of Y₁ and Y₂ is N;
A₁, A₂, A₃, and A₄ are each independently selected from a substituted or unsubstituted aromatic group containing 6 to 30 carbon atoms or a substituted or unsubstituted heteroaromatic group containing 5 to 30 carbon atoms; A₁ and A₂ form a ring or do not form a ring together, and A₃ and A₄ form a ring or do not form a ring together;
when M₁ is B, X₁ and X₂ are each independently selected from electron-donating groups;
when M₁ is N, X₁ and X₂ are each independently selected from electron-withdrawing groups;
when M₂ is B, X₃ and X₄ are each independently selected from electron-donating groups; and
when M₂ is N, X₃ and X₄ are each independently selected from electron-withdrawing groups.

The present application further provides a display panel, including:
a substrate;
a first electrode layer disposed on a side of the substrate;
a second electrode layer disposed on a side of the first electrode layer away from the substrate; and
an organic functional layer disposed between the first electrode layer and the second electrode layer;
in which a material of the organic functional layer includes at least one organic compound described above.

### BRIEF DESCRIPTION OF THE DRAWINGS

FIG. 1 is a schematic structural diagram of a light-emitting element provided by some embodiments of the present application; and
FIG. 2 is a schematic structural diagram of a display panel provided by some embodiments of the present application.

### DETAILED DESCRIPTION

The present application provides an organic compound and a display panel. In order to make the purpose, technical solutions, and effects of the present application clearer and more definite, the following contents will provide a detailed explanation of this disclosure with reference to the drawings and the embodiments of implementation. It should be understood that the embodiments described here are only used to illustrate the present application and are not intended to limit the present application.

In the present application, an aryl group, an aromatic group, and an aromatic ring system have the same meaning and may be interchanged.

In the present application, a heteroaryl group, a heteroaromatic group, and a heteroaromatic ring system have the same meaning and may be interchanged.

In the present application, "substituted" means that a hydrogen atom in a group to be substituted is substituted by a substituent group.

In the present application, a same substituent group at different substituent sites may be independently selected from different groups. For example, if a formula includes multiple R groups, the R groups may be independently selected from different groups.

In the present application, "substituted or unsubstituted" means that a defined group may be substituted or not be substituted. When the defined group is substituted, it can be understood that the defined group may be substituted by one or more substituent R groups. The R groups are each independently selected from, but not limited to, D, T, a cyanoyl group, an isocyanoyl group, a nitro group, a halogen group, a C₁₋₂₀ alkyl group, a heterocyclic group containing 3 to 20 carbon atoms, an aromatic group containing 6 to 20 carbon atoms, a heteroaromatic group containing 5 to 20 carbon atoms, -NR'R", a silyl group, a carbonyl group, an alkoxycarbonyl group, an aryloxycarbonyl group, an aminoformyl group, a haloformyl group, a formyl group, an isocyanate group, a thiocyanate group, an isothiocyanate group, a hydroxyl group, or a trifluoromethyl group, and the groups may further be substituted by acceptable substituent groups in the art. Understandably, R' and R" in the NR'R" are each independently selected from, but not limited to, H, D, T, a cyanoyl group, an isocyanoyl group, a nitro group, a halogen group, a C₁-C₁₀ alkyl group, a heterocyclic group containing 3 to 20 carbon atoms, an aromatic group containing 6 to 20 carbon atoms, or a heteroaromatic group containing 5 to 20 carbon atoms. Preferably, R is selected from, but not limited to, D, a cyanoyl group, an isocyanoyl group, a nitro group, a halogen group, a C₁-C₁₀ alkyl group, a heterocyclic group containing 3-10 carbon atoms, an aromatic group containing 6-20 carbon atoms, a heteroaromatic group containing 5-20 carbon atoms, a silyl group, a carbonyl group, an alkoxycarbonyl group, an aryloxycarbonyl group, an aminoformyl group, a haloformyl group, a formyl group, an isocyanate group, a thiocyanate group, an isothiocyanate group, a hydroxyl group, or a trifluoromethyl group, and the groups may further be substituted by acceptable substituent groups in the art.

In the present application, "the aryl group or the aromatic group" refers to an aromatic hydrocarbon group derived from a basis of an aromatic ring compound removing one hydrogen atom. The aromatic hydrocarbon group may be an aryl group with a single ring, a fused ring aryl group, or a polycyclic aryl group. For a polycyclic ring type, at least one ring is an aromatic ring system. For example, "a substituted or unsubstituted aryl group containing 6 to 40 ring atoms" refers to an aryl group containing 6 to 40 ring atoms, preferably a substituted or unsubstituted aryl group containing 6 to 30 ring atoms, more preferably a substituted or unsubstituted aryl group containing 6 to 18 ring atoms, most preferably a substituted or unsubstituted aryl group containing 6 to 14 ring atoms, and the aryl group is optionally further substituted. Suitable examples of the aryl group or the aromatic group include, but not limited to, a phenyl group, a biphenyl group, a triphenyl group, a naphthyl group, an anthracyl group, a phenanthryl group, a fluoranthenyl group, a triphenylene group, a pyrenyl group, a perylene group, a tetraphenyl group, a fluorenyl group, a diphenyl group, an acenaphthenyl group, and derivatives thereof. Understandably, multiple aryl groups may further be disconnected by short non-aromatic units (for example, a non-hydrogenium atom contenting less than 10%, such as C, N, or O). In particular, an acenaphthene group, a fluorene group, a 9,9-diarylfluorene group, a triarylamine group, or a diaryl ether system may be further included in a definition of the aryl group.

In the present application, "the heteroaryl group or the heteroaromatic group" refers to a basis of an aryl group with at least one carbon atom substituted by a non-carbon atom, and the non-carbon atom may be N, O, S, or the like. For example, "a substituted or unsubstituted heteroaryl group containing 5 to 40 ring atoms" refers to a heteroaryl group containing 5 to 40 ring atoms, preferably a substituted or unsubstituted heteroaryl group containing 6 to 30 ring atoms, more preferably a substituted or unsubstituted heteroaryl group containing 6 to 18 ring atoms, most preferably a substituted or unsubstituted heteroaryl group containing 6 to 14 ring atoms, and the heteroaryl group is optionally further substituted. Suitable examples of the heteroaryl group or the heteroaromatic group include, but not limited to, a thienyl group, a furyl group, a pyrrolyl group, a diazole group, a triazole group, an imidazolyl group, a pyridinyl group, a bipyridyl group, a pyrimidinyl group, a triazinyl group, an acridinyl group, a pyridazinyl group, a pyrazinyl group, a quinolinyl group, an isoquinolinyl group, a quinazolinyl group, a quinoxalinyl group, a phthalazinyl group, a pyridino pyrimidinyl group, a pyridino pyrazinyl group, a benzothiophenyl group, a benzofuranyl group, an indolyl group, a pyrrolo imidazolyl group, a pyrrolopyrrolyl group, a thiophenopyrrolyl group, a thiophenothiophenyl group, a furanopyrrolyl group, a furanofuranyl group, a thiophenofuranyl group, a benzoisoxazolyl group, a benzoisothiazolyl group, a benzimidazolyl group, an ortho-diazonaphthalyl group, a phenanthridinyl group, a berberine group, a quinazolinketone group, a dibenzothiophenyl group, a dibenzofuranyl group, a carbazolyl group, and derivatives thereof.

In the present application, "an alkyl group" refers to a linear alkyl group, a branched alkyl group, and/or a cyclic alkyl group. The number of carbon atoms in the alkyl group may range from 1 to 50, 1 to 30, 1 to 20, 1 to 10, or 1 to 6. The term containing the alkyl group, such as "a C₁₋₉ alkyl group", refers to an alkyl group containing 1 to 9 carbon atoms. The C₁₋₉ alkyl group is independently selected from a C₁ alkyl group, a C₂ alkyl group, a C₃ alkyl group, a C₄ alkyl group, a C₅ alkyl group, a C₆ alkyl group, a C₇ alkyl group, a C₈ alkyl group, or a C₉ alkyl group at each occurrence. Examples of the alkyl group include, but not limited to, methyl, ethyl, n-propyl, isopropyl, n-butyl, sec-butyl, tert-butyl, isobutyl, 2-ethylbutyl, 3,3-dimethylbutyl, n-pentyl, isopentyl, neopentyl, tert-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butyhexyl, cyclohexyl, 4-methylcyclohexyl, 4-tert-butylcyclohexyl, n-heptyl, 1-methylheptyl, 2,2-dimethylheptyl, 2-ethylheptyl, 2-butyl-heptyl, n-octyl, tert-octyl, 2-ethyloctyl, 2-butyl-octyl, 2-hexyl-octyl, 3,7-dimethyloctyl, cyclooctyl, n-nonyl, n-decanyl, an adamantine group, 2-ethyldecyl, 2-butyldecyl 2-hexyldecyl, 2-octyldecyl, n-undecyl, n-dodecyl, 2-ethyldodecyl 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyeicosyl, 2-hexyleicosyl, 2-octyleicosyl, n-heneicosyl, n-docosyl, n-tricosyl, n-tetracosyl, n-pentacosyl, n-hexacosyl, n-heptacosyl, n-octacosyl, n-nonacosyl, n-triacontyl, and the like.

In the present application, "*" connected to a single bond indicates a linking site or a fusing site.

In the present application, when a linking site in a group is not specified, it means that any of connectable sites in the group may be selected as the linking site.

In the present application, when a fusing site in a group is not specified, it means that any of fusible sites in the group may be selected as the fusing site. Preferably, two or more adjacent sites in the group form a fusing site.

In the present application, when there are more than one substituent groups with the same symbol on the same group, the substituent groups may be the same or different. For example, in formula 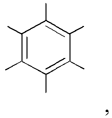 six R groups of a benzene ring may be the same or different.

In the present application, a single bond connected to a substituent group and penetrated a corresponding ring indicates that the substituent group may be connected to any site of the ring. For example, 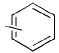 means that R may be connected to any substituent site of the benzene ring, and 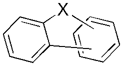 means that 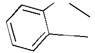 may be connected to any substituent site of 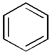 to form a union ring.

According to the present application, a cyclic alkyl group and a cycloalkyl group have the same meaning and may be interchanged.

In the present application, "adjacent groups" refer to two groups absent of substitutable sites between the two groups.

In the present application, "two adjacent R₁, R₃, or R₅ forming a ring with each other" indicate that a ring system is formed by connecting two adjacent R₁, R₃, or R₅ together with the groups to which they attach, and the ring system may be selected from an aliphatic hydrocarbon ring, an aliphatic heterocyclic ring, an aromatic hydrocarbon ring, or an aromatic heterocyclic ring. Preferably, the ring system may be

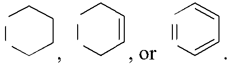

In the present application, the highest occupied molecular orbital (HOMO) and lowest unoccupied molecular orbital (LUMO) energy levels may be measured through methods having the photoelectric effect, such as X-ray photoelectron spectroscopy (XPS), ultraviolet photoelectron spectroscopy (UPS), or cyclic voltammetry (CV). In addition, quantum chemical methods, such as density functional theory (DFT), are also effective methods for calculating molecular orbital energy levels.

Luminescence lifetimes of organic materials can be obtained through technologies such as time related single photon counting (TCSPC).

By providing the organic compound with a multi-resonance effect and elongating the distance between B and B atoms in the organic compound and/or introducing N atom(s) to break conjugation to weaken para-electronic coupling, the present application improves properties of the organic compound, thereby improving luminescence efficiency and color purity of display panels using the organic compound, and prolonging service life of display panels.

Embodiments of the present application provide an organic compound having a structure represented by formula (1): 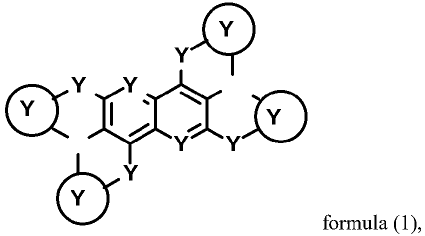
in which M₁ and M₂ are each independently selected from B or N;
Y₁ and Y₂ are each independently selected from C or N, and at least one of Y₁ and Y₂ is N;
A₁, A₂, A₃, and A₄ are each independently selected from a substituted or unsubstituted aromatic group containing 6 to 30 carbon atoms or a substituted or unsubstituted heteroaromatic group containing 5 to 30 carbon atoms; A₁ and A₂ form a ring or do not form a ring together, and A₃ and A₄ form a ring or do not form a ring together;
when M₁ is B, X₁ and X₂ are each independently selected from electron-donating groups;
when M₁ is N, X₁ and X₂ are each independently selected from electron-withdrawing groups;
when M₂ is B, X₃ and X₄ are each independently selected from electron-donating groups; and
when M₂ is N, X₃ and X₄ are each independently selected from electron-withdrawing groups.

The organic compound has a multi-resonance effect. The multi-resonance effect refers to the atomic level of localization and separation of frontier molecular orbitals in polycyclic aromatic hydrocarbons with special structures due to the special positions of electron-donating groups and electron-withdrawing groups, and is conducive to realizing extremely narrow emission spectrum and suppressing the intensity of shoulder peaks at long wavelengths in the emission spectrum, thereby improving color purity of luminescence of the organic compound. Moreover, due to the relatively fixed molecular skeleton, the emission spectrum of the multi-resonance molecules is difficult to adjust, making it difficult to obtain materials that can meet commercial needs. The organic compound has the structure represented by formula (1), by elongating the distance between B and B atoms in the organic compound and/or introducing N atom(s) to break conjugation, para-electronic coupling in the organic compound can be weakened, which is conducive to blue shift of the maximum emission wavelength of the organic compound and reducing energy loss caused by non-radiative transition, thereby improving luminescence performances of the organic compound, and thus improving luminescence efficiency and color purity of display panels using the organic compound, and prolonging service life of display panels.

By providing the organic compound with the multi-resonance effect and elongating the distance between B and B atoms in the organic compound and/or introducing N atom(s) to break conjugation to weaken para-electronic coupling, the present application improves properties of the organic compound, thereby improving luminescence efficiency and color purity of display panels using the organic compound, and prolonging service life of display panels.

In some embodiments, M₁ and M₂ are each independently selected from B or N, and M₁ and M₂ may be the same or different. Preferably, M₁ and M₂ are the same, that is, both M₁ and M₂ are B or N. When both M₁ and M₂ are B, the distance between B and B atoms in the organic compound is elongated to weaken the para-electronic coupling, which is conducive to the blue shift of the maximum emission wavelength of the organic compound and reducing the non-radiative transition caused by the connected groups used for making the organic compound blue shift. Moreover, when both M₁ and M₂ are B, the significant electronegativity difference of B atom or N atom over C atom in the organic compound is conducive to enhancing the multi-resonance effect of the organic compound, thereby further narrowing the emission spectrum of the organic compound and suppressing the intensity of shoulder peaks at long wavelengths in the emission spectrum, and thus further improving the color purity of luminescence of the organic compound. When both M₁ and M₂ are N, the intramolecular conjugation of the organic compound is broken to weaken the para-electronic coupling, which is conducive to the blue shift of the maximum emission wavelength of the organic compound and reducing the non-radiative transition caused by the connected groups used for making the organic compound blue shift.

In some embodiments, when M₁ is B, X₁ and X₂ are each independently selected from electron-donating groups; and when M₂ is B, X₃ and X₄ are each independently selected from electron-donating groups.

In some embodiments, when M₁ is N, X₁ and X₂ are each independently selected from electron-withdrawing groups; and when M₂ is N, X₃ and X₄ are each independently selected from electron-withdrawing groups.

In some embodiments, the electron-donating groups are groups that increase electron cloud density on the benzene ring when one or more H atoms in the benzene ring are substituted; and the electron-withdrawing groups are groups that decrease electron cloud density on the benzene ring when one or more H atoms in the benzene ring are substituted.

In some embodiments, when M₁ is B, X₁ and X₂ are each independently selected from a group consisting of N-R₁, O, and S; and when M₂ is B, X₃ and X₄ are each independently selected from a group consisting of N-R₁, O, and S.

When M₁ is N, X₁ and X₂ are each independently selected from a group consisting of B-R₁, a carbonyl group, a sulfonyl group, and a sulfoxide group; and when M₂ is N, X₃ and X₄ are each independently selected from a group consisting of B-R₁, a carbonyl group, a sulfonyl group, and a sulfoxide group.

R₁ is independently selected from H, D, T, a substituted or unsubstituted linear alkyl group containing 1 to 5 carbon atoms, a substituted or unsubstituted branched alkyl group containing 3 to 12 carbon atoms, a substituted or unsubstituted cycloalkyl group containing 3 to 12 carbon atoms, a substituted or unsubstituted aromatic group containing 6 to 12 carbon atoms, or a substituted or unsubstituted heteroaromatic group containing 5 to 12 carbon atoms at each occurrence.

In some embodiments, when R₁ is selected from a substituted or unsubstituted linear alkyl group containing 1 to 5 carbon atoms, R₁ is preferably selected from an unsubstituted linear alkyl group containing 1 to 5 carbon atoms, for example, methyl, ethyl, n-propyl, n-butyl, n-pentyl, or the like.

In some embodiments, when R₁ is selected from a substituted or unsubstituted branched alkyl group containing 3 to 12 carbon atoms or a substituted or unsubstituted cycloalkyl group containing 3 to 12 carbon atoms, R₁ is selected from an unsubstituted branched alkyl group containing 3 to 12 carbon atoms or an unsubstituted cycloalkyl group containing 3 to 12 carbon atoms. R₁ is preferably selected from an unsubstituted alkyl group containing 3 to 5 carbon atoms, for example, isopropyl, sec-butyl, tert-butyl, isobutyl, isopentyl, neopentyl, tert-pentyl, or the like. Further, R₁ is preferably selected from a cycloalkyl group containing 3 to 6 carbon atoms, for example, cyclopropyl, cyclobutyl, cyclopentyl, cyclohexyl, or the like.

In some embodiments, when R₁ is selected from a substituted or unsubstituted aromatic group containing 6 to 15 carbon atoms, R₁ is preferably selected from a substituted or unsubstituted aromatic group containing 6 or 12 ring atoms or a substituted or unsubstituted aryl group containing 10 ring atoms, for example, an unsubstituted phenyl, an unsubstituted naphthyl, 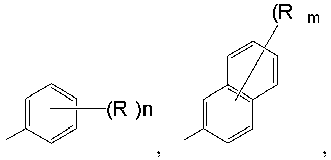 or the like, in which "*" indicates a linking site, n is selected from 1, 2, 3, 4, or 5, R₇ is selected from D, T, an alkyl group containing 1 to 6 carbon atoms (for example, a linear alkyl group containing 1 to 6 carbon atoms, a branched alkyl group containing 3 to 6 carbon atoms, or a cycloalkyl group containing 3 to 6 carbon atoms), or a phenyl group, m is selected from any integer ranging from 1 to 9, and R₈ is selected from D, T, methyl, ethyl, or the like.

In some embodiments, when R₁ is selected from a substituted or unsubstituted heteroaromatic group containing 5 to 15 carbon atoms, R₁ is preferably selected from an unsubstituted heteroaromatic group containing 5 to 12 carbon atoms. For example, R₁ may be selected from pyridinyl, carbazolyl, benzofuranyl, a benzothiophene group, dibenzofuranyl, a dibenzothiophene group, naphthyl, quinolyl, an anaphthyridine group, or the like.

In some embodiments, A₁, A₂, A₃, and A₄ are each independently selected from a substituted or unsubstituted aromatic group containing 6 to 30 carbon atoms. Preferably, A₁, A₂, A₃, and A₄ are each independently selected from a substituted or unsubstituted aromatic group containing 6 to 15 carbon atoms, for example, 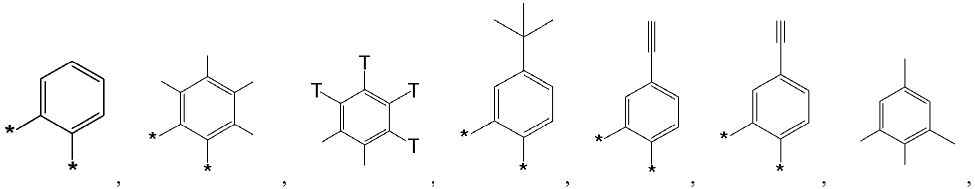 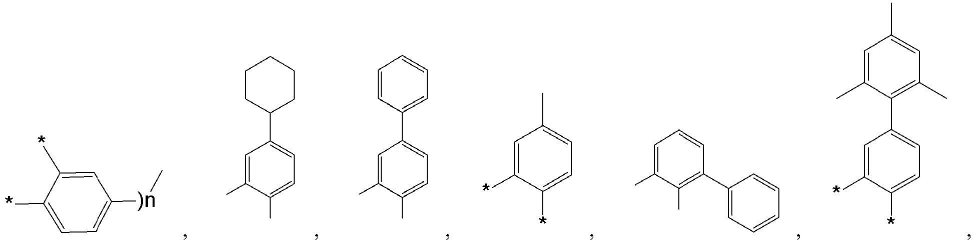 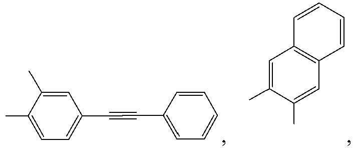 or the like.

In some embodiments, A₁, A₂, A₃, and A₄ are each independently selected from a substituted or unsubstituted heteroaromatic group containing 5 to 30 carbon atoms. Preferably, A₁, A₂, A₃, and A₄ are each independently selected from a substituted or unsubstituted heteroaromatic group containing 5 to 18 carbon atoms, for example, 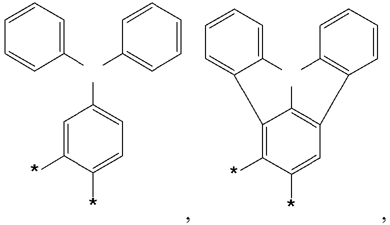 or the like.

In some embodiments, at least one of A₁ to A₄ adjacent to R₁ is bridged with R₁ through a single bond or a first bridging group. For example, when X₁ is N-R₁ or B-R₁, A₁ may be bridged with R₁ through a single bond or the first bridging group; when X₂ is N-R₁ or B-R₁, A₂ may be bridged with R₁ through a single bond or the first bridging group; when X₃ is N-R₁ or B-R₁, A₃ may be bridged with R₁ through a single bond or the first bridging group; and when X₄ is N-R₁ or B-R₁, A₄ may be bridged with R₁ through a single bond or the first bridging group.

In some embodiments, the first bridging group is independently selected from a group consisting of N-R₂, CR₃R₄, SiR₅R₆, O, S, Se, Te, a carbonyl group, a sulfonyl group, and a sulfoxide group at each occurrence. R₂, R₃, R₄, R₅, and R₆ are each independently selected from H, D, T, a substituted or unsubstituted linear alkyl group containing 1 to 5 carbon atoms, a substituted or unsubstituted branched alkyl group containing 3 to 6 carbon atoms, a substituted or unsubstituted cycloalkyl group containing 3 to 6 carbon atoms, a substituted or unsubstituted aromatic group containing 6 to 15 carbon atoms, or a substituted or unsubstituted heteroaromatic group containing 5 to 15 carbon atoms.

In some embodiments, R₂, R₃, R₄, R₅, and R₆ are each independently selected from H, D, T, a substituted or unsubstituted linear alkyl group containing 1 to 5 carbon atoms, a substituted or unsubstituted branched alkyl group containing 1 to 5 carbon atoms, a substituted or unsubstituted aromatic group containing 6 to 10 carbon atoms, or a substituted or unsubstituted heteroaromatic group with 6 to 12 carbon atoms at each occurrence.

In some embodiments, R₂, R₃, R₄, R₅, and R₆ are each independently selected from methyl or phenyl at each occurrence.

In some embodiments, the first bridging group is independently selected from a group consisting of 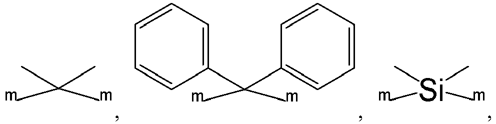 O, S, Se, Te, a carbonyl group, a sulfonyl group, and a sulfoxide group at each occurrence.

In some embodiments, A₁ and A₂ are bridged through a single bond or a second bridging group.

In some embodiments, A₃ and A₄ are bridged through a single bond or a third bridging group.

In some embodiments, the second bridging group and the third bridging group are each independently selected from a group consisting of a substituted or unsubstituted linear alkyl group containing 1 to 5 carbon atoms, a branched alkyl group, SiR₉R₁₀, O, S, Se, Te, a carbonyl group, a sulfonyl group, and a sulfoxide group at each occurrence.

In some embodiments, R₉ and R₁₀ are each independently selected from a substituted or unsubstituted linear alkyl group containing 1 to 5 carbon atoms, a branched alkyl group, or a substituted or unsubstituted aromatic group containing 6 to 10 carbon atoms.

In some embodiments, both R₉ and R₁₀ are methyl.

In some embodiments, the second bridging group and the third bridging group are each independently selected from a group consisting of 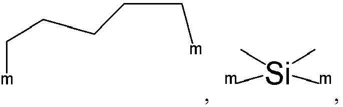 O, S, Se, Te, a carbonyl group, a sulfonyl group, and a sulfoxide group at each occurrence.

In some embodiments, M₁ and M₂ are the same, and both Y₁ and Y₂ are N, which is conducive to further breaking the conjugation and weakening the para-electronic coupling, thereby facilitating the blue shift of the maximum emission wavelength of the organic compound and reduces energy loss caused by the non-radiative transition, and thus further improving the luminescence performances of the organic compound, enhancing luminescence efficiency and color purity of display panels using the organic compound, and prolonging service life of display panels.

In some embodiments, M₁ and M₂ are the same, A₁, A₂, A₃, and A₄ are the same, and X₁, X₂, X₃, and X₄ are the same, which facilitate the synthesis of the organic compound and reduce manufacturing cost.

In some embodiments, when M₁ and M₂ are the same, A₁, A₂, A₃, and A₄ are the same, and X₁, X₂, X₃, and X₄ are the same, the organic compound has a structure represented by formula (1-1) or formula (1-2):

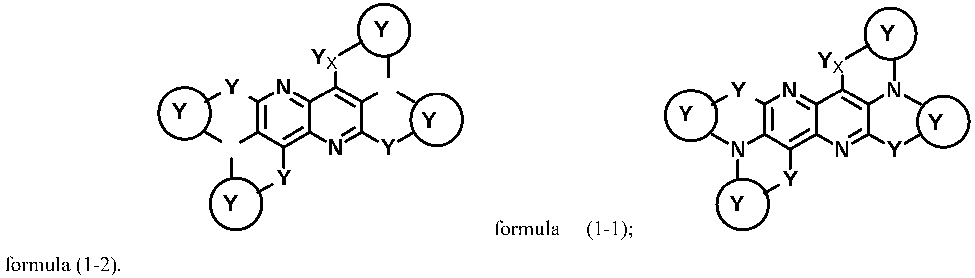

In the formula (1-1), M₁ and M₂ are the same, both which are B. In the formula (1-2), M₁ and M₂ are the same, both which are N. In the formula (1-1) and the formula (1-2), Y₁ and Y₂ are the same, both which are N.

In some embodiments, the organic compound is selected from a group consisting of the following compounds:

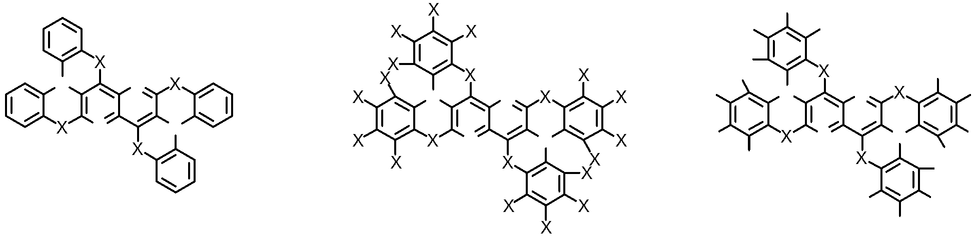

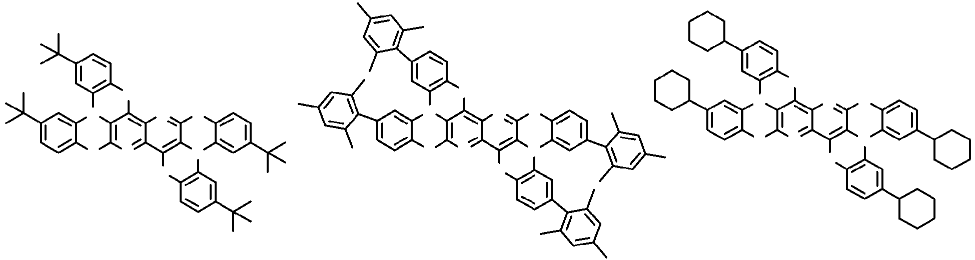

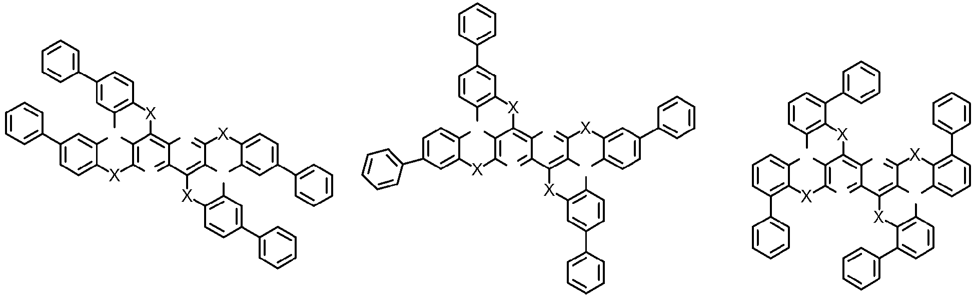

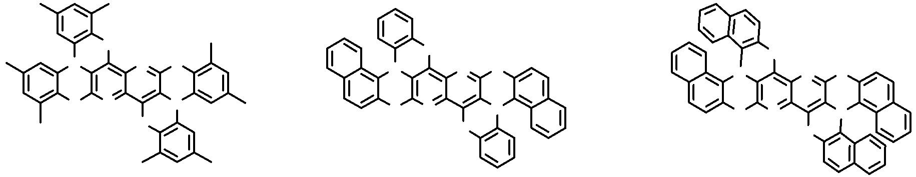

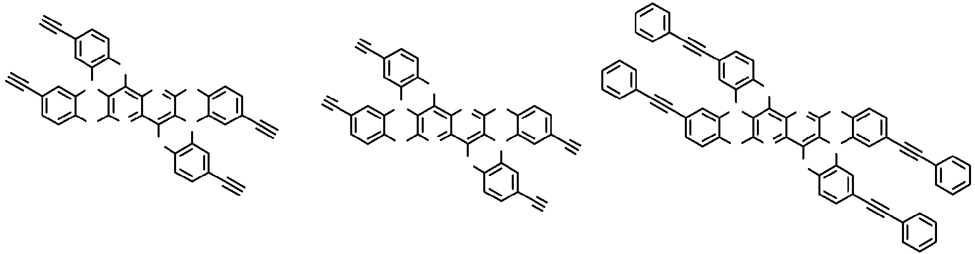

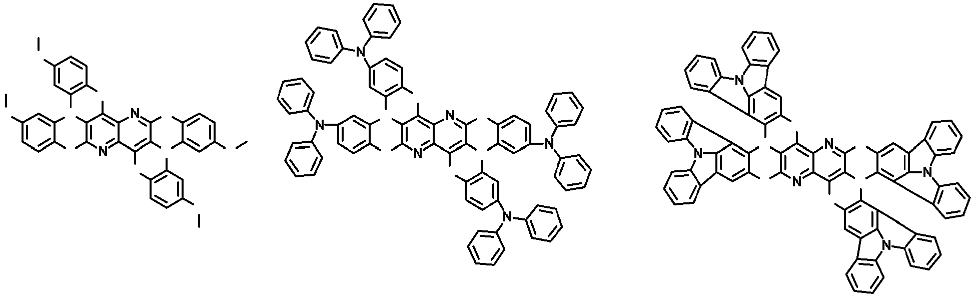

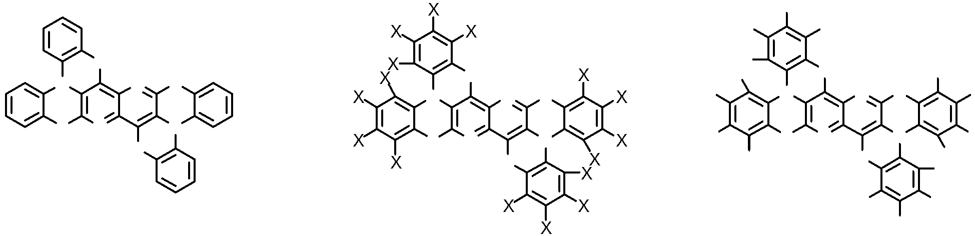

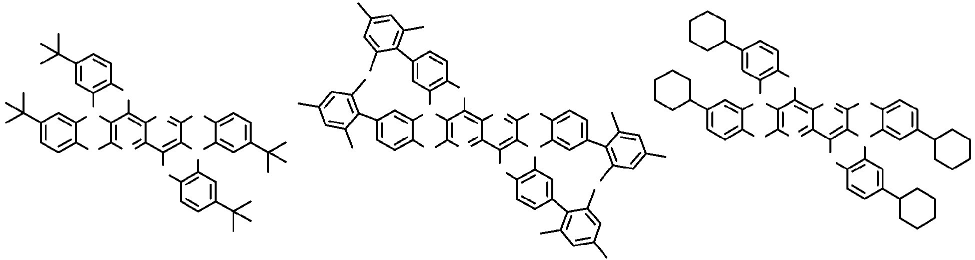

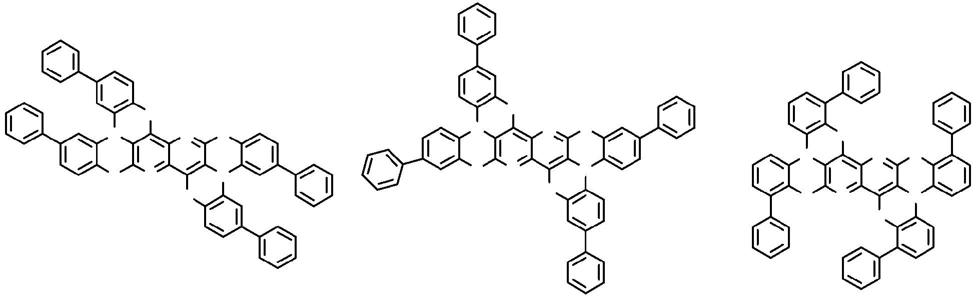

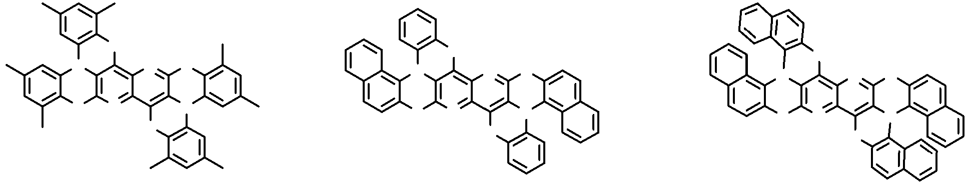

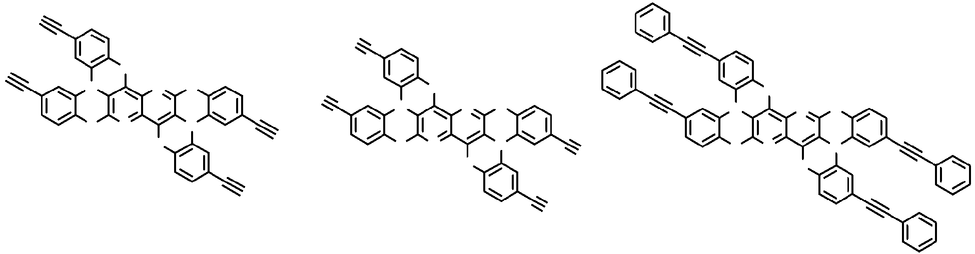

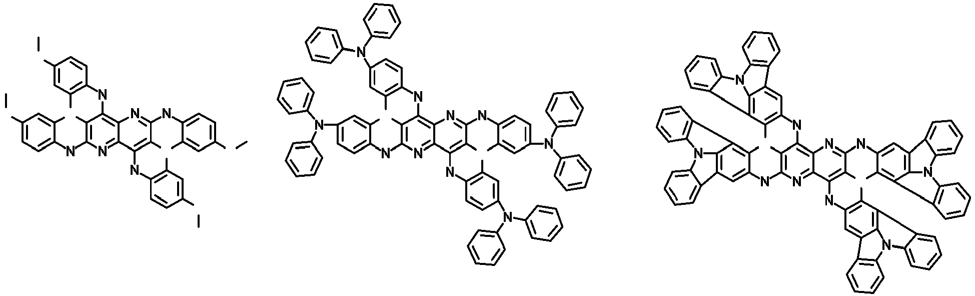

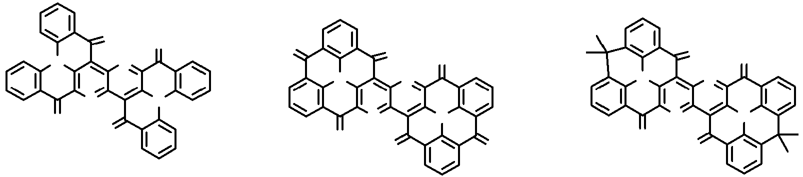

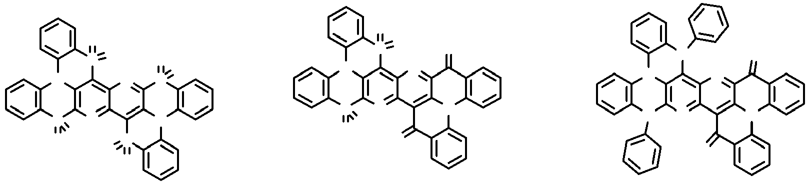

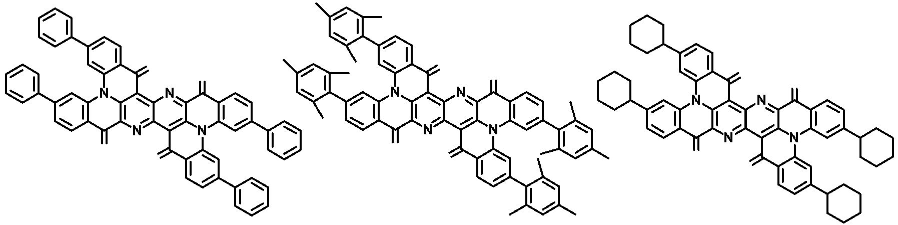

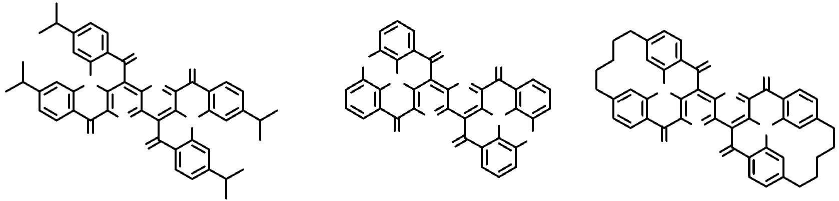

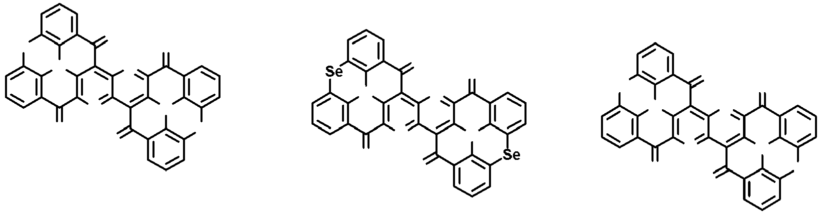

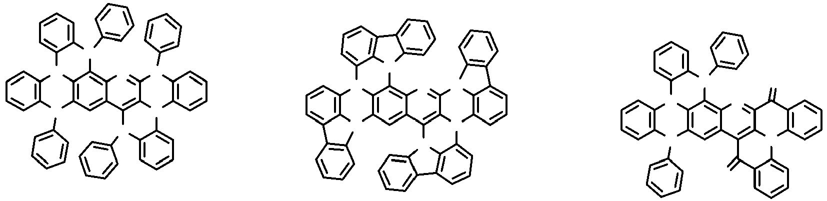

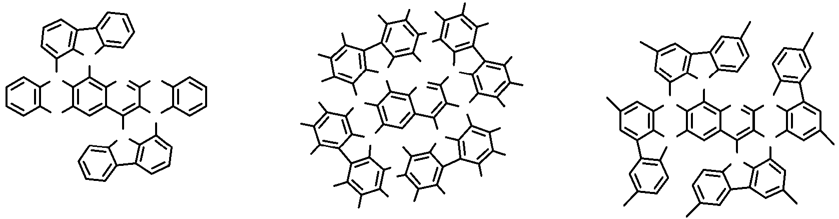

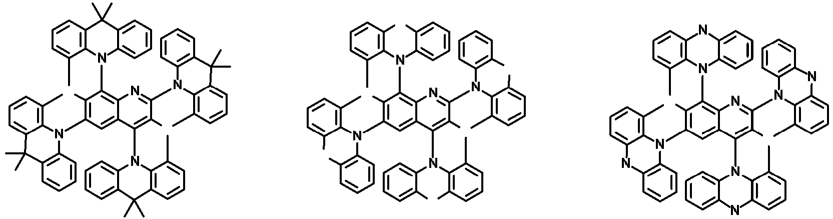

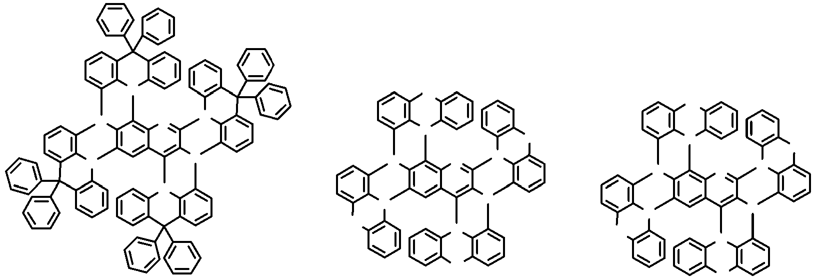

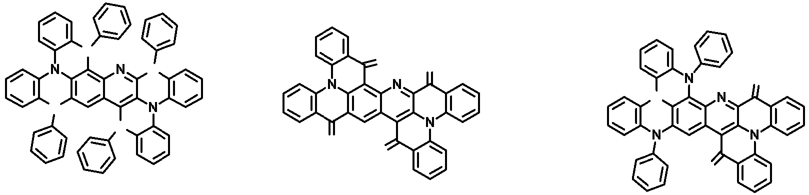

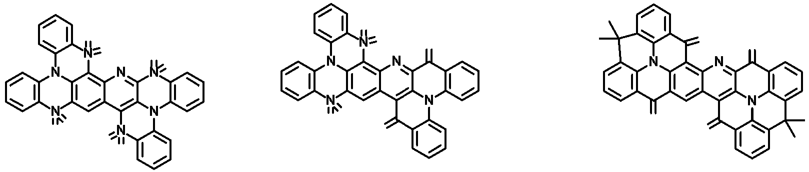

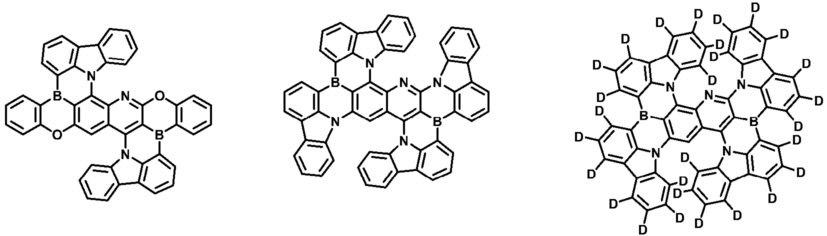

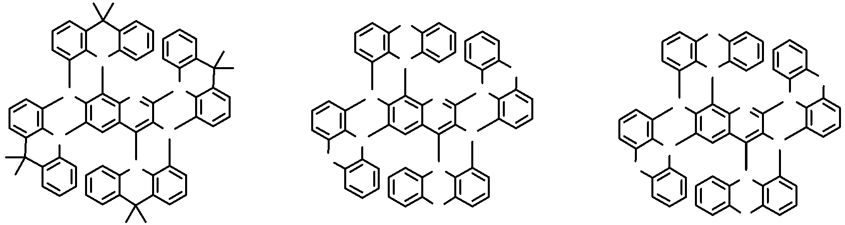

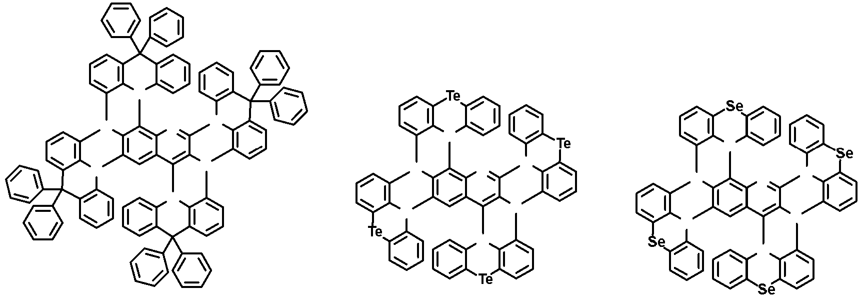

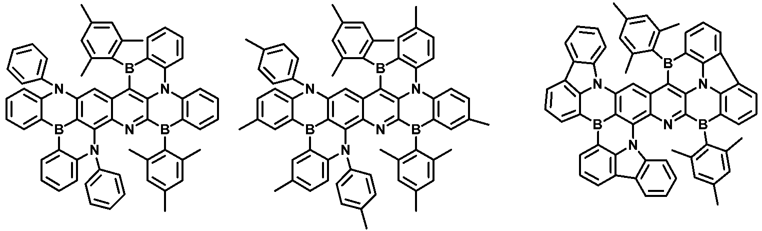

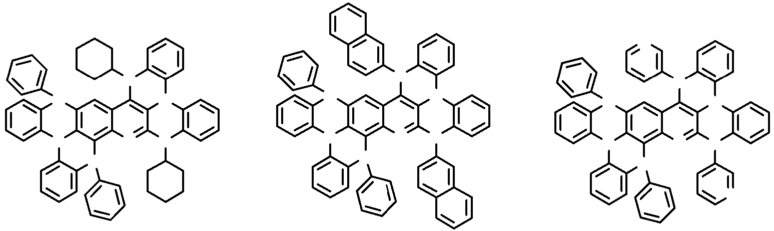

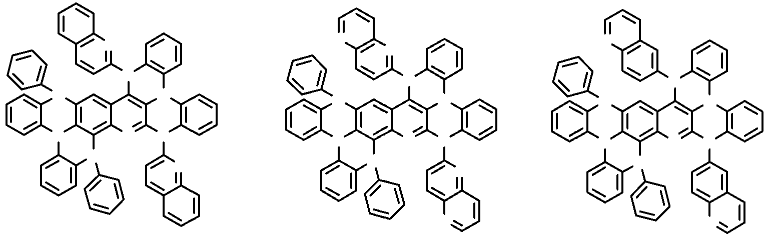

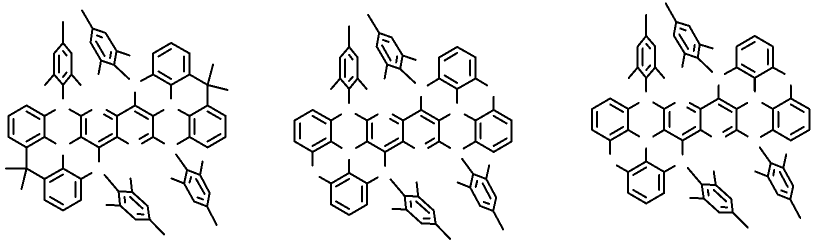

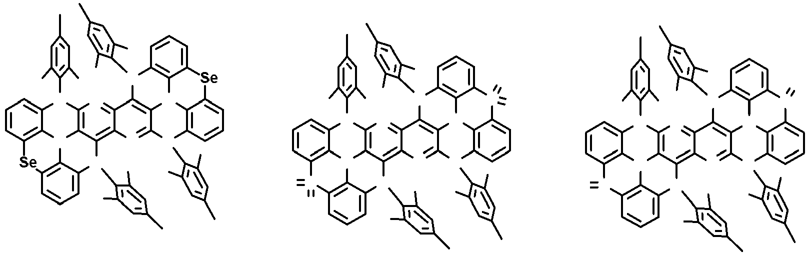

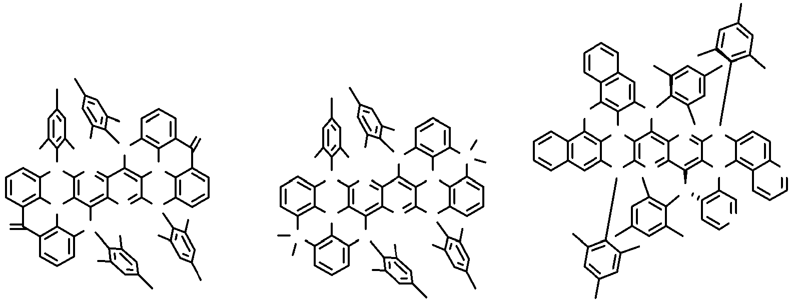

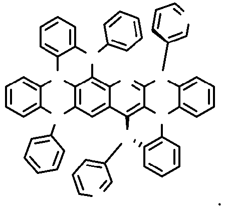

In some embodiments, the highest occupied molecular orbital energy level of the organic compound is greater than or equal to -6.0 eV and greater than or equal to -5.0 eV, for example, -5.9 eV, -5.8 eV, -5.7 eV, -5.6 eV, -5.5 eV, -5.4 eV, -5.3 eV, -5.2 eV, -5.1 eV, or the like.

In some embodiments, the lowest unoccupied molecular orbital energy level of the organic compound is greater than or equal to -4.0 eV and greater than or equal to -3.0 eV, for example, -3.9 eV, -3.8 eV, -3.7 eV, -3.6 eV, -3.5 eV, -3.4 eV, -3.3 eV, -3.2 eV, -3.1 eV, or the like.

In some embodiments, a dilute solution of the organic compound at room temperature has the emission spectrum ranging from 380 nanometers to 800 nanometers. Preferably, the dilute solution of the organic compound at room temperature has the emission spectrum ranging from 480 nanometers to 620 nanometers. The dilute solution of the organic compound may be obtained by dissolving the organic compound in a solvent that can dissolve the organic compound, such as toluene. The dilute solution may have a concentration ranging from 10⁻⁴ mol/L to 10⁻⁶ mol/L, for example, 10⁻⁴ mol/L.

In some embodiments, the dilute solution of the organic compound has a full width at half maxima of the emission spectrum of less than 60 nanometers at room temperature, for example, 20 nanometers, 30 nanometers, 40 nanometers, 50 nanometers, or the like, which is conducive to improving the color purity of luminescence of the organic compound.

In some embodiments, the dilute solution of the organic compound has a maximum emission peak value of the emission spectrum ranging from 480 nanometers to 580 nanometers at room temperature. Preferably, the dilute solution of the organic compound has a maximum emission peak value of the emission spectrum ranging from 520 nanometers to 575 nanometers at room temperature, which is conducive to obtaining green light-emitting materials with higher color purity.

In some embodiments, solid-state films made of the organic compound have fluorescence life ranging from 1 microsecond to 10 milliseconds. For example, when substituent groups in the organic compound are heavy atoms, the fluorescence life of the organic compound can be effectively extended to the millisecond level.

By providing the organic compound with the multi-resonance effect and elongating the distance between B and B atoms in the organic compound and/or introducing N atom(s) to break conjugation to weaken para-electronic coupling, the embodiments of the present application improves the properties of the organic compound, thereby improving luminescence efficiency and color purity of display panels using the organic compound, and prolonging service life of display panels.

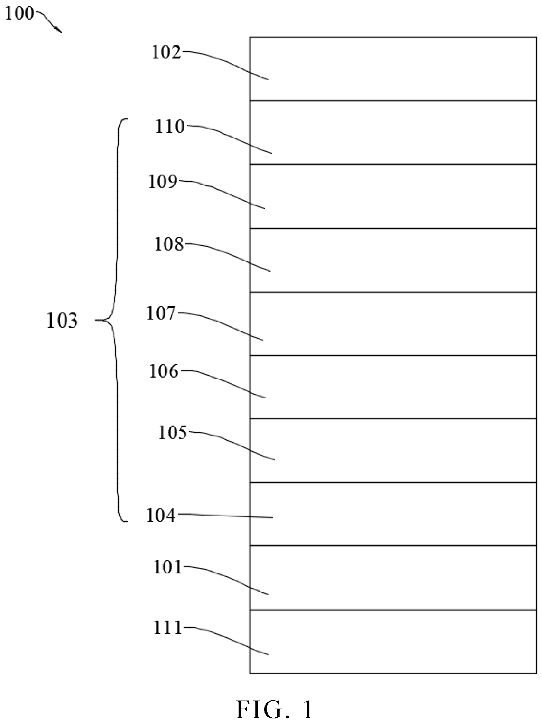

Referring to FIG. 1, the present application further provides a light-emitting element 100 that includes a pair of electrodes and an organic functional layer 103. The electrodes include a first electrode layer 101 and a second electrode layer 102. The organic functional layer 103 is disposed between the first electrode layer 101 and the second electrode layer 102. A material of the organic functional layer 103 includes one or more organic compounds as described above. The first electrode layer 101 may be an anode, and the second electrode layer 102 may be a cathode.

In some embodiments, the light-emitting element 100 may be used as an organic light-emitting diode, an organic photovoltaic cell, an organic light-emitting cell, an organic field-effect transistor, an organic light-emitting field-effect transistor, an organic laser, an organic spintronic device, an organic sensor, or an organic plasmon emitting diode, preferably an organic light-emitting diode, an organic light-emitting cell, or an organic light-emitting field-effect transistor.

In some embodiments, the light-emitting element 100 can be used in various electronic devices, such as display panels, lighting devices, light sources, or the like.

In some embodiments, the organic functional layer 103 includes at least a light-emitting layer 107. Preferably, the organic functional layer 103 includes a hole injection layer 104, a hole transport layer 105, an electron blocking layer 106, the light-emitting layer 107, a hole blocking layer 108, an electron transport layer 109, and an electron injection layer 110.

In some embodiments, the light-emitting element 100 may be a blue light-emitting element, a green light-emitting element, or a red light-emitting element.

In some embodiments, the light-emitting layer 107 may include a host material and a guest material, and the guest material is one or more of the organic compounds described above. When the guest material is selected from one or more of the organic compounds mentioned above, the light-emitting element 100 is preferably a green light-emitting element, and the light-emitting element 100 has a light-emitting wavelength within the wavelength range of green light.

In some embodiments, the host material includes a fused aromatic derivative or a heteroaromatic compound.

In some embodiments, the host material includes a fused aromatic ring derivative, a compound containing heterocyclic rings, or the like, for example, at least one of an anthracene derivative, a pyrene derivative, a naphthalene derivative, a pentabenzene derivative, a phenanthrene compound, a fluoranthene compound, a carbazole derivative, a dibenzofuran derivative, a ladder type furan compound, and a pyrimidine derivative.

In some embodiments, when the light-emitting layer 107 includes the host material and the guest material, a ratio of the host material to the guest material ranges from 99:1 to 70:30 by mass, for example, 90:10, 85:15, 80:20, 75:25, or the like. Preferably, when the light-emitting layer 107 is composed of the host material and the guest material, the ratio of the host material to the guest material ranges from 99:1 to 90:10 by mass, for example, 98:2, 97:3, 96:4, 95:5, 93:7, 92:8, or the like. The guest material is dispersed in the host material, and the ratio of the host material to the guest material ranges from 99:1 to 70:30 by mass, which is conducive to suppressing the crystallization of the light-emitting layer 107 and the concentration quenching caused by high concentration of the guest material, thereby improving luminescence efficiency of the light-emitting element 100.

In some embodiments, the light-emitting layer 107 further includes a sensitizer. The sensitizer may be selected from a group consisting of a phosphorescence material and a thermally activated delayed fluorescence (TADF) material. By adding the sensitizer, triplet state excitons in the light-emitting element 100 can be effectively utilized, so that energy of the triplet state excitons can be transferred from the sensitizer to the organic compound, which further improves luminescence efficiency and enhances stability of the light-emitting element 100.

In some embodiments, the highest occupied molecular orbital energy level of the sensitizer is greater than or equal to -6.0 eV and greater than or equal to -5.0 eV, for example, -5.9 eV, -5.8 eV, -5.7 eV, -5.6 eV, -5.5 eV, -5.4 eV, -5.3 eV, -5.2 eV, -5.1 eV, or the like.

In some embodiments, the lowest unoccupied molecular orbital energy level of the sensitizer is greater than or equal to -4.0 eV and greater than or equal to -3.0 eV, for example, -3.9 eV, -3.8 eV, -3.7 eV, -3.6 eV, -3.5 eV, -3.4 eV, -3.3 eV, -3.2 eV, -3.1 eV, or the like.

By matching the highest occupied molecular orbital energy level of the sensitizer with the highest occupied molecular orbital energy level of the organic compound, and matching the lowest unoccupied molecular orbital energy level of the sensitizer with the lowest unoccupied molecular orbital energy level of the organic compound, the use of the sensitizer facilitates the injection of carriers.

In some embodiments, a ratio among the organic compound, the sensitizer, and the host compound in the light-emitting layer 107 ranges from 1 to 3:10 to 30:167 to 189 by mass, for example, 2:20:178 by mass.

In some embodiments, the sensitizer is preferably a phosphorescence material with phosphorescence emission. The phosphorescence material has a maximum emission peak value of emission spectrum ranging from 480 nanometers to 580 nanometers and phosphorescence life ranging from 1 microsecond to 100 milliseconds.

In some embodiments, the sensitizer is preferably a phosphorescence material containing at least one of metal atoms consisting of Ir, Pd, Pt, Cu, Ag, and Au.

In some embodiments, the sensitizer is selected from a group consisting of the following compounds:

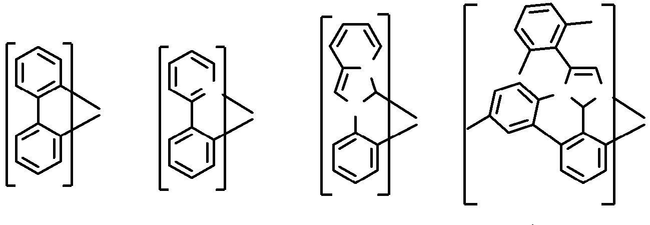

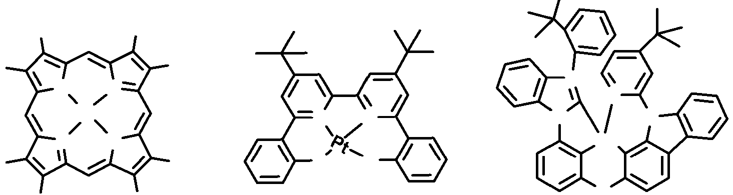

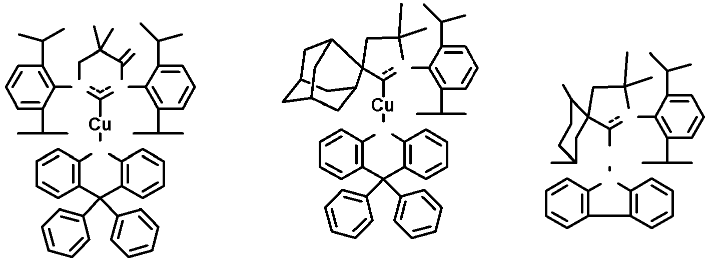

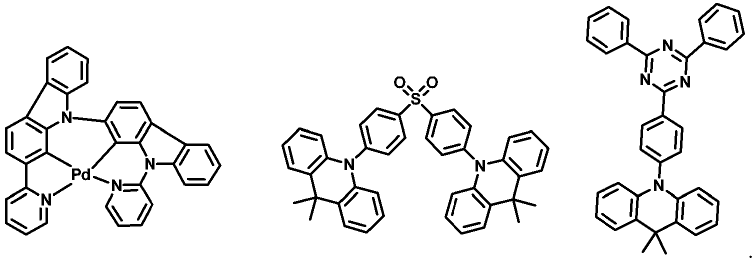

In some embodiments, the anode is an electrode used for injecting holes, and the holes in the anode can be injected into the organic functional layer 103. For example, the holes in the anode can be injected into the hole injection layer 104, the hole transport layer 105, or the light-emitting layer 107. A material of the anode may include at least one of a conductive metal, a conductive metal oxide, and a conductive polymer. The material of the anode includes, but not limited to, at least one of aluminum (Al), copper (Cu), aurum (Au), argentum (Ag), magnesium (Mg), ferrum (Fe), cobalt (Co), nickel (Ni), manganese (Mn), palladium (Pd), platinum (Pt), indium tin oxide (ITO), aluminum doped zinc oxide (AZO), and other suitable anode materials known in the art, which can be easily chose and used by those skilled in the art. The material of the anode can be deposited using any suitable technology, such as a suitable physical vapor deposition method including RF magnetron sputtering, vacuum thermal evaporation, electron beam (e-beam), or the like. In some embodiments, the anode is a patterned structure, such as a patterned ITO conductive substrate that can be commercially available and used to prepare the light-emitting element of the present application.

In some embodiments, the cathode is an electrode used for injecting electrons, and the electrons in the cathode can be easily injected into the organic functional layer. For example, the electrons in the cathode can be easily injected into the electron injection layer 110, the electron transport layer 109, or the light-emitting layer 107. A material of the cathode may include at least one of a conductive metal and a conductive metal oxide. All materials that can be used in the cathode of an organic electronic device may be used as the material of the cathode of the organic electronic device of the present application. The material of the cathode includes, but not limited to, at least one of Al, Au, Ag, calcium (Ca), barium (Ba), Mg, LiF/Al, MgAg alloy, BaF₂/Al, Cu, Fe, Co, Ni, Mn, Pd, Pt, ITO, and the like. The material of the cathode can be deposited using any suitable technology, such as a suitable physical vapor deposition method including RF magnetron sputtering, vacuum thermal evaporation, electron beam (e-beam), or the like.

In some embodiments, the hole injection layer 104 is used for promoting an injection of holes from the anode to the light-emitting layer 107. The hole injection layer 104 includes a hole injection material that is a material used to receive holes injected from a positive electrode at low voltages. Preferably, HOMO energy level of the hole injection material is between work function of the material of the anode and HOMO energy level of a functional material of film layers on a side of the hole injection layer away from the anode (such as the hole transport material of the hole transport layer 105). The hole injection material includes, but not limited to, at least one of metalloporphyrin, oligothiophene, an organic material based on arylamine, an organic material based on hexacyano hexaazabenzophenanthrene, an organic material based on quinacridone, an organic material based on perylene, anthraquinone, a conductive polymer based on polyaniline, polythiophene, and the like.

In some embodiments, the hole transport layer 105 is used for transmitting holes to the light-emitting layer 107. The hole transport layer 105 includes a hole transport material used to receive holes transmitted from the anode or the hole injection layer 104 and transmit the holes to the light-emitting layer 107. The hole transport material is a material known in the art to have high hole mobility. The hole transport material includes, but not limited to, at least one of an aryl amine-based organic material, an organic material based on carbazolyl group, a conductive polymer, a block copolymer with both conjugated and non-conjugated portions, and the like.

In some embodiments, the electron transport layer 109 is used for transmitting electrons. The electron transport layer 109 includes an electron transport material used to receive electrons injected from a negative electrode and transmit the electrons to the light-emitting layer 107. The electron transport material has high electron mobility known in the art. The electron transport material includes, but not limited to, at least one of an Al-based complex of 8-hydroxyquinoline, a complex containing Alq₃, an organic radical compound, a hydroxyflavone metal complex, 8-hydroxyquinoline lithium (LiQ), and a compound based on benzimidazole.

In some embodiments, the electron injection layer 110 is used for injecting electrons. The electron injection layer 110 includes an electron injection material. Preferably, the electron injection material has ability to transmit electrons, and can achieve an effect of injecting electrons from a negative electrode and an excellent effect of injecting electrons into the light-emitting layer 107 or the light-emitting material, preventing excitons generated by the light-emitting layer 107 from transferring to the hole injection layer 104. Further, the electron injection material has excellent ability to form a thin film. The electron injection material includes, but not limited to, at least one of 8-hydroxyquinoline Lithium (LiQ), fluorenone, anthraquinone dimethane, biphenylquinone, thiopyran dioxide, azole, diazole, triazole, imidazole, perylene tetracarboxylic acid, fluorenylidene methane, anthrone, or the like, and their derivatives, metal coordination compounds, nitrogen-containing 5-membered ring derivatives.

In some embodiments, the hole blocking layer 108 is used for blocking holes from reaching the negative electrode, and generally formed under the same condition as the hole injection layer 104. The hole blocking layer 108 includes a hole blocking material, which includes, but not limited to, at least one of a diazole derivative, a triazole derivative, a phenanthroline derivative, BCP, an aluminum complex, and the like.

In some embodiments, the light-emitting element 100 further includes an element substrate 111, and the first electrode 101, the hole injection layer 104, the hole transport layer 105, the electron blocking layer 106, the light-emitting layer 107, the electron transport layer 109, the electron injection layer 110, and the second electrode 102 are sequentially stacked on the element substrate 111. The element substrate 111 may be a transparent substrate or an opaque substrate. When the element substrate 111 is a transparent substrate, a transparent light-emitting element can be prepared. The element substrate 111 may be a rigid substrate or a flexible substrate with elasticity. A material of the element substrate 111 may include, but not limited to, plastic, polymer, metal, a semiconductor wafer, a glass, or the like. Preferably, the element substrate 111 includes at least one smooth surface for forming the anode.

Exemplary preparation methods of the organic compounds provided by the present application are illustrated in the following exemplary examples 1 to 10.

### Example 1

Synthesis of Organic Compound M₁ (Molecular formula: C₆₈H₅₆B₂N₂O₄, 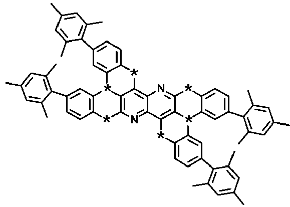 )

Synthesis Route of Organic Compound M1 is as follows:

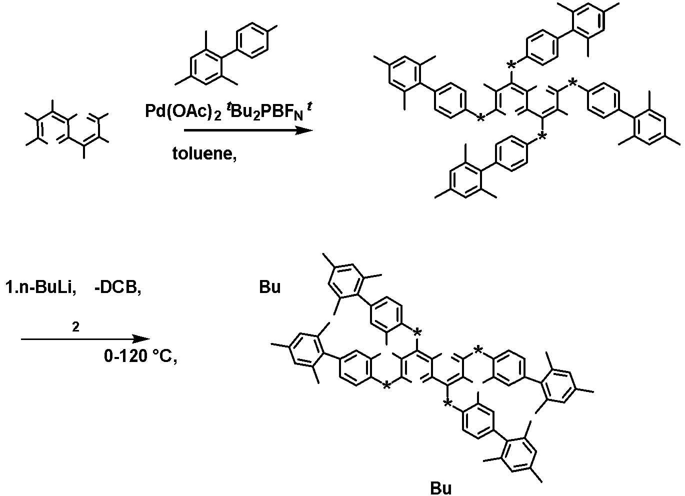

### Synthesis Steps of Organic Compound M1 are as follows:

### Synthesis of Intermediate 1-a:

2,3,4,6,7,8-hexabromo-1,5-naphthyridine (5.8 g, 10 mmol), 4'-hydroxyl-2,4,6-trimethylbiphenyl (8.9 g, 41 mmol), palladium acetate (0.4 g, 2 mmol), tri-tert-butylphosphine tetrafluoroborate (1.8 g, 6 mmol), and lithium tert-butanol (4.0 g, 50 mmol) were added to 1000 mL of a two-necked flask, and 400 mL of dried toluene was added to the reaction solution under argon protection. Then the reaction solution was reacted at 90 °C for 24 hours under argon protection. After cooling to room temperature, the reaction solution was poured into 300 mL of saturated sodium chloride aqueous solution, extracted with dichloromethane, and washed with saturated sodium chloride aqueous solution. Then organic phases were combined and concentrated to obtain the intermediate 1-a (9.2 g, yield of 82%) by column chromatography. Measured value of MS(ESI)(m/z)[M]⁺ was 1129.2.

### Synthesis of Organic Compound M1:

The intermediate 1-a (3.3 g, 3 mmol) and ultra-dried ortho-dichlorobenzene (30 mL) were added to 100 mL of a single-necked flask, and stirred at -30 °C for 15 minutes under anhydrous oxygen-free atmosphere. N-butyl lithium (0.21 g, 3.0 mmol) was slowly added to the reaction solution dropwise at low temperature, then the reaction solution was raised to 15 °C and reacted for 2 hours. Subsequently, the reaction solution was cooled to -30 'C, boron tribromide (1.50 g, 6.0 mmol) was quickly added therein, heated to 60 °C and reacted for 3 hours. Then N, N-diisopropylethylamine (0.77 g, 6.0 mmol) was quickly added to the reaction solution in an ice bath condition. After white smoke disappeared, the reaction solution was raised to 120 °C and reacted for 1 hour. After the reaction was completed, the mixed solution was cooled to room temperature, and methanol was added dropwise in an ice bath condition to quench the reaction. The mixed solution was spun to remove organic solvents to obtain a black solid. The black solid was extracted three times with dichloromethane and water, then organic phases were collected and concentrated to obtain the organic compound M1 (0.44 g, yield of 15%) by column chromatography. Element analysis results (using Perkin Elmer 2400, CHN mode) were as follows: theoretical values of C of 82.77, H of 5.72, and N of 2.84, and measured values of C of 82.24, H of 5.33, and N of 2.21. Theoretical value of MS(ESI)(m/z)[M]⁺ was 986.83, and measured value of MS(ESI)(m/z)[M]⁺ was 986.22.

### Example 2

Synthesis of Organic Compound M2 (Molecular formula: C₃₆H₁₆N₄O₄, 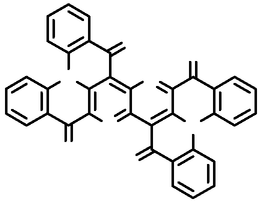 )

### Synthesis Route of Organic Compound M2 is as follows:

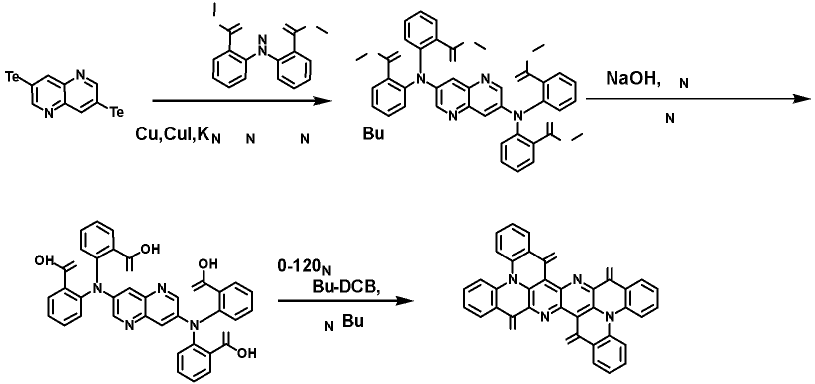

### Synthesis Steps of Organic Compound M2 are as follows:

### Synthesis of Intermediate 2-a:

3,7-dibromo-1,5-naphthalidine (2.85 g, 10 mmol), 2-{[2-(methoxycarbonyl)phenyl]amino}methylbenzoate (8.5 g, 30 mmol), Cu powder (0.4 g, 6 mmol), CuI (0.6 g, 3 mmol), potassium carbonate (5.0 g, 36 mmol), and 200 mL of dibutyl ether were added to 500 mL of a two-necked flask, and reacted at 150 °C for 48 hours under argon protection. After cooling to room temperature, 300 mL of saturated sodium chloride aqueous solution was added to the reaction solution, and extracted several times with dichloromethane. Then organic phases were combined and concentrated to obtain the intermediate 2-a (4.7 g, yield of 72%) by column chromatography. Measured value of MS(ESI)(m/z)[M]⁺ was 696.76.

### Synthesis of Intermediate 2-b:

The intermediate 2-a (3.5 g, 5 mmol), sodium hydroxide (2.0 g, 50 mmol), 50 mL of distilled water, and 50 mL of dioxane were added to 500 mL of a single-necked flask, and reacted at 100 °C for 12 hours under reflux. After cooling to room temperature, concentrated hydrochloric acid was added to the reaction solution until pH of the reaction solution was about 5.0. Then the reaction solution was treated with suction filtration to obtain a filter cake. The filter cake was washed with water to obtain the intermediate 2-b (3.0 g, yield of 95%). Measured value of MS(ESI)(m/z)[M]⁺ was 640.39.

### Synthesis of Organic Compound M2:

The intermediate 2-b (2.6 g, 4 mmol) and dichloromethane (80 mL) were added to a three-necked flask, two drops of DMF were added, and then thionyl chloride (1.3 mL, 17.6 mmol) was added dropwise. The reaction solution was reacted at 60 °C for 5 hours under reflux. Then aluminum chloride (2.3 g, 17.6 mmol) was added to the reaction solution and continue the reflux for 12 hours. After cooling to room temperature, the reaction solution was added to 300 mL of sodium hydroxide aqueous solution (1 M) dropwise and extracted with dichloromethane. Organic phases were concentrated to obtain the organic compound M2 (1.5 g, yield of 67%) by column chromatography. Element analysis results (using Perkin Elmer 2400, CHN mode) were as follows: theoretical values of C of 80.04, H of 3.36, and N of 8.49, and measured values of C of 80.32, H of 3.44, and N of 8.21. Theoretical value of MS(ESI)(m/z)[M]⁺ was 568.12, and measured value of MS(ESI)(m/z)[M]⁺ was 568.37.

### Example 3

Synthesis of Organic Compound M3 (Molecular formula: C₆₈H₅₂B₂N₆, 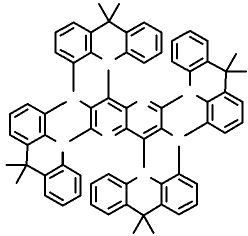 )

Synthesis Route of Organic Compound M3 is as follows:

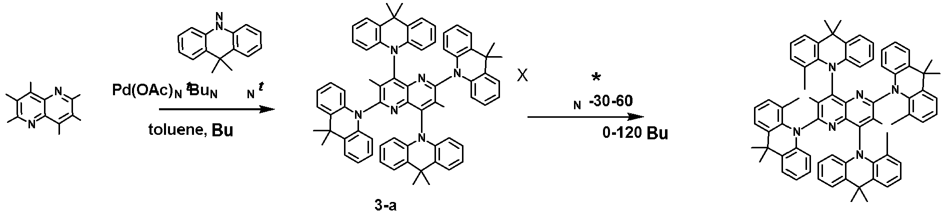

### Synthesis Steps of Organic Compound M3 are as follows:

### Synthesis of Intermediate 3-a:

9,9-dimethyl-9,10-dihydroacridine was substituted for 4'-hydroxyl-2,4,6-trimethylbiphenyl in the synthesis of intermediate 1-a to obtain the intermediate 3-a (6.8 g, yield 62%) using the same method. Measured value of MS(ESI)(m/z)[M]⁺ was 1117.0.

### Synthesis of Organic Compound M3:

The intermediate 3-a (3.3 g, 3 mmol) and ultra-dried ortho-dichlorobenzene (30 mL) were added to 100 mL of a single-necked flask, and stirred at -30 °C for 15 minutes under anhydrous oxygen-free atmosphere. N-butyl lithium (0.21 g, 3.0 mmol) was slowly added to the reaction solution dropwise at low temperature, then the reaction solution was raised to 15 °C and reacted for 2 hours. Subsequently, the reaction solution was cooled to -30 °C, boron tribromide (1.50 g, 6.0 mmol) was quickly added therein, heated to 60 °C and reacted for 3 hours. Then N, N-diisopropylethylamine (0.77 g, 6.0 mmol) was quickly added to the reaction solution in an ice bath condition. After white smoke disappeared, the reaction solution was raised to 120 °C and reacted for 1 hour. After the reaction was completed, the mixed solution was cooled to room temperature, and methanol was added dropwise in an ice bath condition to quench the reaction. The reaction solution was spun to remove organic solvents to obtain a black solid. The black solid was extracted three times with dichloromethane and water, then organic phases were collected and concentrated to obtain the organic compound M3 (0.29 g, yield of 10%) by column chromatography. Element analysis results (using Perkin Elmer 2400, CHN mode) were as follows: theoretical values of C of 83.78, H of 5.38, and N of 8.62, and measured values of C of 83.46, H of 5.12, and N of 8.66. Theoretical value of MS(ESI)(m/z)[M]⁺ was 974.44, and measured value of MS(ESI)(m/z)[M]⁺ was 974.73.

### Example 4

Synthesis of Organic Compound M4 (Molecular formula: C₁₀₈H₆₈B₂N₆, 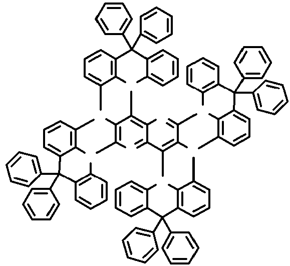 )

Synthesis Route of Organic Compound M4 is as follows:

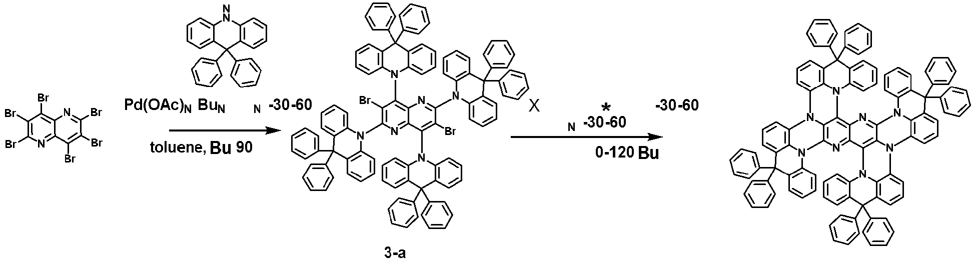

### Synthesis Steps of Organic Compound M4 are as follows:

### Synthesis of Intermediate 4-a:

10H-spiro[acr-9,9'-fluorene] was substituted for 4'-hydroxyl-2,4,6-trimethylbiphenyl in the synthesis of intermediate 1-a to obtain the intermediate 4-a (8.5 g, yield of 53%) using the same method. Measured value of MS(ESI)(m/z)[M]⁺ was 1613.2.

### Synthesis of Organic Compound M4:

The intermediate 4-a (4.8 g, 3 mmol) and ultra-dried ortho-dichlorobenzene (30 mL) were added to 100 mL of a single-necked flask, and stirred at -30 °C for 15 minutes under anhydrous oxygen-free atmosphere. N-butyl lithium (0.21 g, 3.0 mmol) was slowly added to the reaction solution dropwise at low temperature, then the reaction solution was raised to 15 °C and reacted for 2 hours. Subsequently, the reaction solution was cooled to -30 °C, boron tribromide (1.50 g, 6.0 mmol) was quickly added therein, heated to 60 °C and reacted for 3 hours. Then N, N-diisopropylethylamine (0.77 g, 6.0 mmol) was quickly added to the reaction solution in an ice bath condition. After white smoke disappeared, the reaction solution was raised to 120 °C and reacted for 1 hour. After the reaction was completed, the mixed solution was cooled to room temperature, and methanol was added dropwise in an ice bath condition to quench the reaction. The reaction solution was spun to remove organic solvents to obtain a black solid. The black solid was extracted three times with dichloromethane and water, then organic phases were collected and concentrated to obtain the organic compound M4 (0.22 g, yield of 5%) by column chromatography. Element analysis results (using Perkin Elmer 2400, CHN mode) were as follows: theoretical values of C of 88.16, H of 4.66, and N of 5.71, and measured values of C of 88.42, H of 4.48, and N of 5.37. Theoretical value of MS(ESI)(m/z)[M]⁺ was 1471.4, and measured value of MS(ESI)(m/z)[M]⁺ was 1471.3.

### Example 5

Synthesis of Organic Compound M5 (Molecular formula: C₅₆D₂₈B₂N₆, 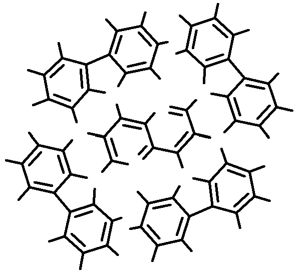 )

Synthesis Route of Organic Compound M5 is as follows:

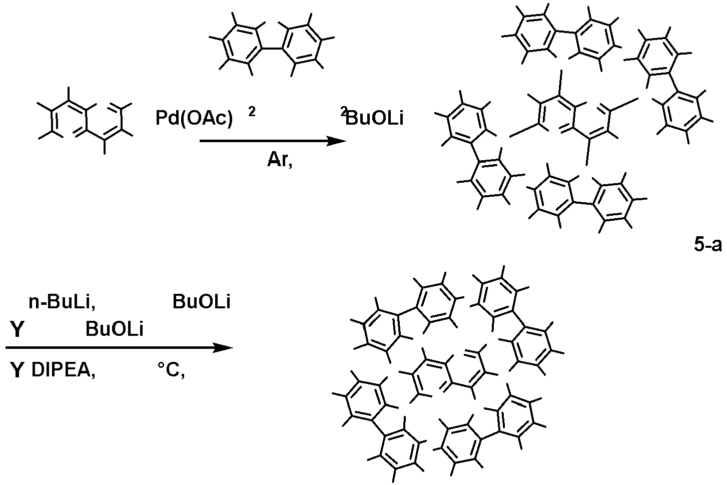

### Synthesis Steps of Organic Compound M5 are as follows:

### Synthesis of Intermediate 5-a:

Deuterated carbazole was substituted for 4'-hydroxyl-2,4,6-trimethylbiphenyl in the synthesis of intermediate 1-a to obtain the intermediate 5-a (7.0 g, yield of 72%) using the same method. Measured value of MS(ESI)(m/z)[M]⁺ was 981.2.

### Synthesis of Organic Compound M5:

The intermediate 4-a (2.9 g, 3 mmol) and ultra-dried ortho-dichlorobenzene (30 mL) were added to 100 mL of a single-necked flask, and stirred at -30 °C for 15 minutes under anhydrous oxygen-free atmosphere. N-butyl lithium (0.21 g, 3.0 mmol) was slowly added to the reaction solution dropwise at low temperature, then the reaction solution was raised to 15 °C and reacted for 2 hours. Subsequently, the reaction solution was cooled to -30 °C, boron tribromide (1.50 g, 6.0 mmol) was quickly added therein, heated to 60 °C and reacted for 3 hours. Then N, N-diisopropylethylamine (0.77 g, 6.0 mmol) was quickly added to the reaction solution in an ice bath condition. After white smoke disappeared, the reaction solution was raised to 120 °C and reacted for 1 hour. After the reaction was completed, the mixed solution was cooled to room temperature, and methanol was added dropwise in an ice bath condition to quench the reaction. The reaction solution was spun to remove organic solvents to obtain a black solid. The black solid was extracted three times with dichloromethane and water, then organic phases were collected and concentrated to obtain the organic compound M5 (0.43 g, yield of 17%) by column chromatography. Element analysis results (using Perkin Elmer 2400, CHN mode) were as follows: theoretical values of C of 80.58, H of 6.76, and N. 10.07, and measured values of C of 80.79, H of 6.99, and N of 10.33. Theoretical value of MS(ESI)(m/z)[M]⁺ was 834.67, and measured value of MS(ESI)(m/z)[M]⁺ was 834.33.

### Example 6

Synthesis of Organic Compound M6 (Molecular formula: C₄₄H₂₂B₂N₄O₂, 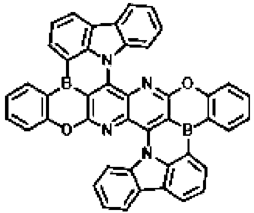 )

Synthesis Route of Organic Compound M6 is as follows:

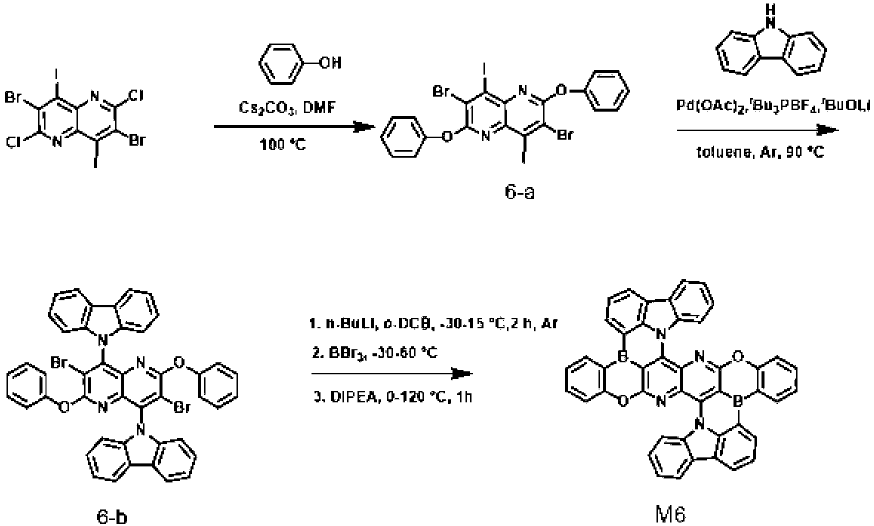

### Synthesis Steps of Organic Compound M6 are as follows:

### Synthesis of Intermediate 6-a:

2,6-dichloro-3,7-dibromo-4,8-diiodio-1,5-naphthyridine (12.2 g, 20 mmol), phenol (4.2 g, 44 mmol), and cesium carbonate (20 g, 60 mmol) were added to 500 mL of a two-necked flask, and 300 mL of dried DMF was added to the reaction solution under argon protection. Then the reaction solution was reacted at 90 °C for 24 hours under argon protection. After cooling to room temperature, the reaction solution was poured into 1 L of saturated sodium chloride aqueous solution, extracted with dichloromethane, and washed with saturated sodium chloride aqueous solution. Then organic phases were combined and concentrated to obtain the intermediate 6-a (11.2 g, yield of 77%) by column chromatography. Measured value of MS(ESI)(m/z)[M]⁺ was 723.93.

### Synthesis of Intermediate 6-b:

The intermediate 6-a (7.2 g, 10 mmol), carbazole (8.9 g, 21 mmol), palladium acetate (0.4 g, 2 mmol), tri-tert-butylphosphine tetrafluoroborate (1.8 g, 6 mmol), and lithium tert-butanol (2.4 g, 30 mmol) were added to 100 mL of a two-necked flask, and 100 mL of dried toluene was added to the reaction solution under argon protection. Then the reaction solution was reacted at 90 °C for 24 hours under argon protection. After cooling to room temperature, the reaction solution was poured into 300 mL of saturated sodium chloride aqueous solution, extracted with dichloromethane, and washed with saturated sodium chloride aqueous solution. Then organic phases were combined and concentrated to obtain the intermediate 1-a (6.3 g, yield of 79%) by column chromatography. Measured value of MS(ESI)(m/z)[M]⁺ was 802.56.

### Synthesis of Organic Compound M6:

The intermediate 6-b (2.4 g, 3 mmol) and ultra-dried ortho-dichlorobenzene (30 mL) were added to 100 mL of a single-necked flask, and stirred at -30 °C for 15 minutes under anhydrous oxygen-free atmosphere. N-butyl lithium (0.21 g, 3.0 mmol) was slowly added to the reaction solution dropwise at low temperature, then the reaction solution was raised to 15 °C and reacted for 2 hours. Subsequently, the reaction solution was cooled to -30 °C, boron tribromide (1.50 g, 6.0 mmol) was quickly added therein, heated to 60 °C and reacted for 3 hours. Then N, N-diisopropylethylamine (0.77 g, 6.0 mmol) was quickly added to the reaction solution in an ice bath condition. After white smoke disappeared, the reaction solution was raised to 120 °C and reacted for 1 hour. After the reaction was completed, the mixed solution was cooled to room temperature, and methanol was added dropwise in an ice bath condition to quench the reaction. The reaction solution was spun to remove organic solvents to obtain a black solid. The black solid was extracted three times with dichloromethane and water, then organic phases were collected and concentrated to obtain the organic compound M6 (0.44 g, yield of 22%) by column chromatography. Element analysis results (using Perkin Elmer 2400, CHN mode) were as follows: theoretical values of C of C of 80.04, H of 3.36, and N of 8.49, and measured values of C of 80.07, H of 3.30, and N of 8.52. Theoretical value of MS(ESI)(m/z)[M]⁺ was 660.31, and measured value of MS(ESI)(m/z)[M]⁺ was 660.37.

### Example 7

Synthesis of Organic Compound M7 (Molecular formula: C₄₀H₃₂N₄O₄, 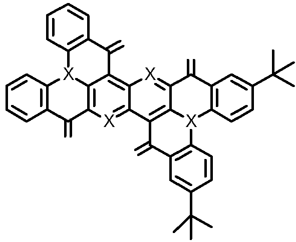 )

Synthesis Route of Organic Compound M7 is as follows:

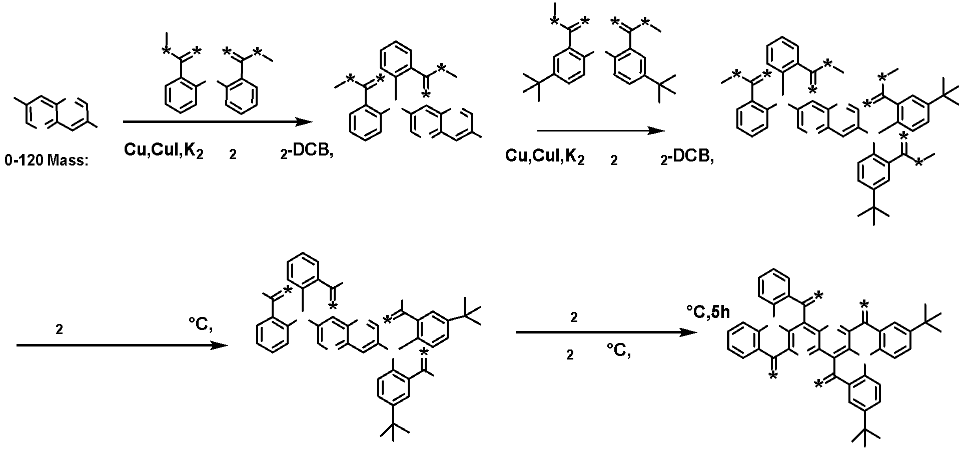

### Synthesis Steps of Organic Compound M7 are as follows:

### Synthesis of Intermediate 7-a:

3,7-dibromo-1,5-naphthalidine (2.85 g, 10 mmol), 2-{[2-(methoxycarbonyl)phenyl]amino}methylbenzoate (2.9 g, 10 mmol), Cu powder (0.2 g, 3 mmol), CuI (0.3 g, 1.5 mmol), potassium carbonate (2.5 g, 18 mmol), and 100 mL of dibutyl ether were added to 500 mL of a two-necked flask, and reacted at 150 °C for 48 hours under argon protection. After cooling to room temperature, 300 mL of saturated sodium chloride aqueous solution was added to the reaction solution, and extracted several times with dichloromethane. Then organic phases were combined and concentrated to obtain the intermediate 7-a (3.1 g, yield of 63%) by column chromatography. Measured value of MS(ESI)(m/z)[M]⁺ was 491.07.

### Synthesis of Intermediate 7-b:

3,7-dibromo-1,5-naphthalidine (2.85 g, 10 mmol), 2-{[2-(methoxycarbonyl)phenyl]amino}methylbenzoate (3.9 g, 10 mmol), Cu powder (0.2 g, 3 mmol), CuI (0.3 g, 1.5 mmol), potassium carbonate (2.5 g, 18 mmol), and dibutyl ether (100 mL) were added to 500 mL of a two-necked flask, and reacted at 150 °C for 48 hours under argon protection. After cooling to room temperature, 300 mL of saturated sodium chloride aqueous solution was added to the reaction solution, and extracted several times with dichloromethane. Then organic phases were combined and concentrated to obtain the intermediate 7-b (5.7 g, yield of 70%) by column chromatography. Measured value of MS(ESI)(m/z)[M]⁺ was 808.17.

### Synthesis of Intermediate 7-c:

The intermediate 7-b (4.0 g, 5 mmol), sodium hydroxide (2.0 g, 50 mmol), 50 mL of distilled water, and 50 mL of dioxane were added to 500 mL of a single-necked flask, and reacted at 100 °C for 12 hours under reflux. After cooling to room temperature, concentrated hydrochloric acid was added to the reaction solution until pH of the reaction solution was about 5.0. Then the reaction solution was treated with suction filtration to obtain a filter cake. The filter cake was washed with water to obtain the intermediate 7-c (3.7 g, yield of 95%). Measured value of MS(ESI)(m/z)[M]⁺ was 752.33.

### Synthesis of Organic Compound M7:

The intermediate 7-c (3.0 g, 4 mmol) and dichloromethane (80 mL) were added to a three-necked flask, two drops of DMF were added, and then thionyl chloride was added (1.3 mL, 17.6 mmol) dropwise. The reaction solution was reacted at 60 °C for 5 hours under reflux. Then aluminum chloride (2.3 g, 17.6 mmol) was added to the reaction solution and continue the reflux for 12 hours. After cooling to room temperature, the reaction solution was added to 300 mL of sodium hydroxide aqueous solution (1 M) dropwise and extracted with dichloromethane. Organic phases were concentrated to obtain the organic compound M7 (1.4 g, yield of 53%) by column chromatography. Element analysis results (using Perkin Elmer 2400, CHN mode) were as follows: theoretical values of C of 77.63, H of 4.74, and N of 8.23, and measured values of C of 77.66, H of 4.71, and N of 8.26. Theoretical value of MS(ESI)(m/z)[M]⁺ was 680.24, and measured value of MS(ESI)(m/z)[M]⁺ was 680.30.

### Example 8

Synthesis of Organic Compound M8 (Molecular formula: C₆₃H₄₉B₃N₄, 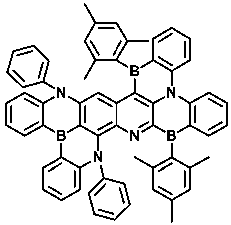 )

Synthesis Route of Organic Compound M8 is as follow:

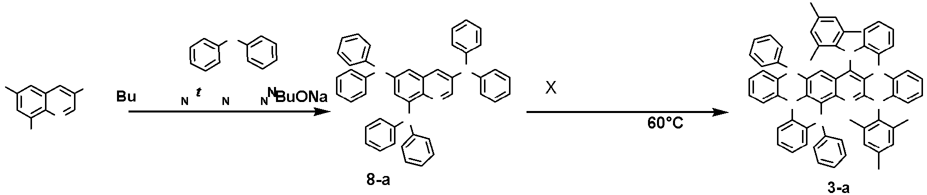

### Synthesis Steps of Organic Compound M8 are as follows:

### Synthesis of Intermediate 8-a:

3,5,7-tribromoquinoline (3.6 g, 10 mmol), diphenylamine (5.9 g, 35 mmol), palladium acetate (0.4 g, 2 mmol), tri-tert-butylphosphine tetrafluoroborate (1.8 g, 6 mmol), and sodium tert-butanol (4.8 g, 50 mmol) were added to 1000 mL of a two-necked flask, and 400 mL of dried toluene was added to the reaction solution under argon protection. Then the reaction solution was reacted at 90 °C for 24 hours under argon protection. After cooling to room temperature, the reaction solution was poured into 300 mL of saturated sodium chloride aqueous solution, extracted with dichloromethane, and washed with saturated sodium chloride aqueous solution. Then organic phases were combined and concentrated to obtain the intermediate 8-a (5.0 g, yield of 80%) by column chromatography. Measured value of MS(ESI)(m/z)[M]⁺ was 630.33.

### Synthesis of Organic Compound M8:

The intermediate 8-a (1.5 g, 2.3 mmol), triphenylborane (7.3 g, 30 mmol), and ultra-dried ortho-dichlorobenzene (30 mL) were added to 100 mL of a single-necked flask, and 12 g of boron triiodide was added to the reaction solution under anhydrous oxygen-free atmosphere. The reaction solution was raised to 180 °C and reacted for 8 hours. After cooling to the room temperature, THF solution (24 mL, 28.8 mmol) of mesitylene formate reagent was added to the reaction solution, stirred at room temperature for 1 hour, heated and stirred at 60 °C, and reacted for 3 hours. Subsequently, the reaction solution was extracted with toluene and dried with anhydrous magnesium sulfate to obtain the organic compound M8 (0.53 g, yield of 27%) by column chromatography. Element analysis results (using Perkin Elmer 2400, CHN mode) were as follows: theoretical values of C of 84.59, H of 5.52, and N of 6.26, and measured values of C of 84.37, H of 5.35, and N of 6.53. Theoretical value of MS(ESI)(m/z)[M]⁺ was 894.42, and measured value of MS(ESI)(m/z)[M]⁺ was 894.22.

### Example 9

Synthesis of Organic Compound M9 (Molecular formula: C₇₈H₆₂B₄N₆S, 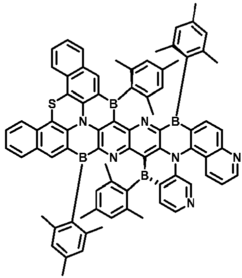 )

Synthesis Route of Organic Compound M9 is as follows:

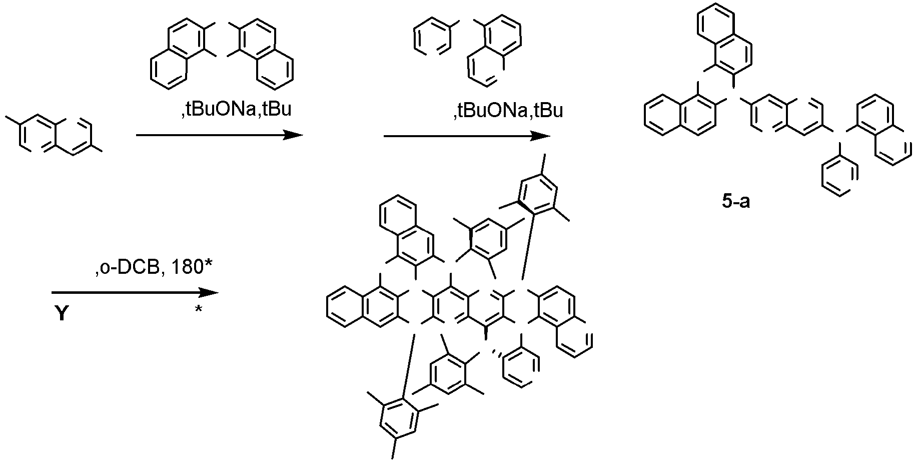

### Synthesis Steps of Organic Compound M9 are as follows:

### Synthesis of Intermediate 9-a:

3,7-dibromo-1,5-naphthyridine (2.85 g, 10 mmol), 7H dibenzo[c,h]phenothiazine (3.0 g, 10 mmol), palladium acetate (0.4 g, 2 mmol), tri-tert-butylphosphine tetrafluoroborate (1.8 g, 6 mmol), and sodium tert-butanol (2.4 g, 25 mmol) were added to 500mL of a two-necked flask, and 200 mL of dried xylene was added to the reaction solution. Then the reaction solution was reacted at 110 °C for 48 hours under argon protection. Then 100mL of toluene dissolved with 5-(3-pyridylamino) quinoline (2.2 g, 10 mmol) was added to the reaction solution and reacted at 110 °C for 48 hours. After cooling to room temperature, 300 mL of saturated sodium chloride aqueous solution was added to the reaction solution, and extracted several times with dichloromethane. Then organic phases were combined and concentrated to obtain the intermediate 9-a (3.1 g, yield of 48%) by column chromatography. Measured value of MS(ESI)(m/z)[M]⁺ was 646.33.

### Synthesis of Organic Compound M9:

The intermediate 9-a (1.5 g, 2.3 mmol), triphenylborane (9.7 g, 40 mmol), and ultra-dried ortho-dichlorobenzene (30 mL) were added to 100 mL of a single-necked flask, and 16 g of boron triiodide was added to the reaction solution under anhydrous oxygen-free atmosphere. The reaction solution was raised to 180 °C and reacted for 8 hours. After cooling to the room temperature, THF solution (48 mL, 57 mmol) of mesitylene formate reagent was added to the reaction solution, stirred at room temperature for 1 hour, heated and stirred at 60 °C, and reacted for 3 hours. Subsequently, the reaction solution was extracted with toluene and dried with anhydrous magnesium sulfate to obtain the organic compound M9 (0.53 g, yield of 20%) by column chromatography. Element analysis results (using Perkin Elmer 2400, CHN mode) were as follows: theoretical values of C of 80.85, H of 5.39, and N of 7.25, and measured values of C of 80.87, H of 5.33, and N of 7.29. Theoretical value of MS(ESI)(m/z)[M]⁺ was 1158.5, and measured value of MS(ESI)(m/z)[M]⁺ was 1158.4.

### Example 10

Synthesis of Organic Compound M10 (Molecular formula: C₅₅H₃₅B₃N₆, 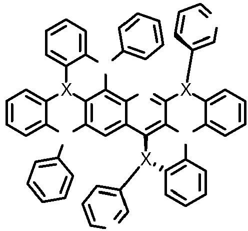 )

Synthesis Route of Organic Compound M10 is as follows:

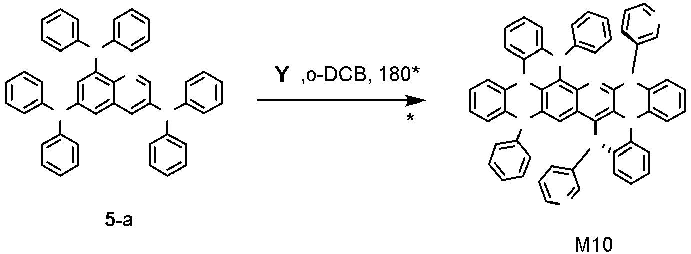

### Synthesis Steps of Organic Compound M10 are as follows:

### Synthesis of Organic Compound M10:

The intermediate 8-a (1.5 g, 2.3 mmol), triphenylborane (7.3 g, 30 mmol), and ultra-dried ortho-dichlorobenzene (30 mL) were added to 100 mL of a single-necked flask, and 12 g of boron triiodide was added to the reaction solution under anhydrous oxygen-free atmosphere. The reaction solution was raised to 180 °C and reacted for 8 hours. After cooling to the room temperature, THF solution (24 mL, 28.8 mmol) of 3-pyridyl format reagent was added to the reaction solution, stirred at room temperature for 1 hour, heated and stirred at 60 °C, and reacted for 3 hours. Subsequently, the reaction solution was extracted with toluene and dried with anhydrous magnesium sulfate to obtain the organic compound M10 (0.39 g, yield of 22%) by column chromatography. Element analysis results (using Perkin Elmer 2400, CHN mode) were as follows: theoretical values of C of 81.32, H of 4.34, and N of 10.35, and measured values of C of 81.36, H of 4.38, and N of 10.27. Theoretical value of MS(ESI)(m/z)[M]⁺ was 812.34, and measured value of MS(ESI)(m/z)[M]⁺ was 812.37.

### Comparative Example 1

Comparative compound 1 is used as a comparative example corresponding to examples 1 to 10, structures of comparative compound 1 (DABNA-1) and comparative compound 2 (R-BN) are as follows:

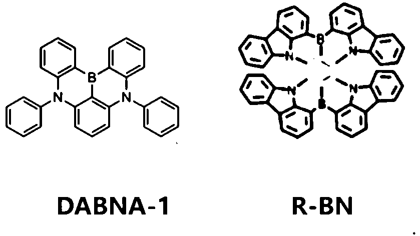

The organic compounds M1 to M10 obtained from examples 1 to 10 and the comparative compounds 1 and 2 are performed on steady-state fluorescence spectroscopy tests (test conditions are as follows: using Hitachi fluorescence spectrometer F-4600 at room temperature, exciting at 360 nm, and using a dilute solution of toluene that has a concentration of 10⁻⁵ mol/L). The test results are shown in table 1.

**Table 1: Maximum emission wavelengths (λ_{Peak}) and full width at half maxima (FWHM) of organic compounds M1 to M10 and comparative compounds 1 and 2**

| | Compound | λ_{Peak} (nm) | FWHM (nm) |
|---|---|---|---|
| Example 1 | Organic compound M1 | 525 | 25 |
| Example 2 | Organic compound M2 | 546 | 32 |
| Example 3 | Organic compound M3 | 538 | 29 |
| Example 4 | Organic compound M4 | 538 | 25 |
| Example 5 | Organic compound M5 | 535 | 22 |
| Example 6 | Organic compound M6 | 527 | 23 |
| Example 7 | Organic compound M7 | 549 | 33 |
| Example 8 | Organic compound M8 | 520 | 22 |
| Example 9 | Organic compound M9 | 562 | 34 |
| Example 10 | Organic compound M10 | 527 | 27 |
| Comparative Example 1 | Comparative compound 1 | 462 | 33 |
| Comparative Example 2 | Comparative compound 2 | 662 | 38 |

According to the results in table 1, it can be seen that, compared with comparative compounds 1 and 2, organic compounds M1 to M10 obtained in examples 1 to 10 of the present application have maximum emission wavelengths ranging from 525 nm to 562 nm, which are within the range of green light. Moreover, organic compounds M1 to M10 have full widths at half maxima of the emission spectra ranging from 22 nm to 34 nm, which are extremely narrow, making it conducive to the use of green light-emitting materials with higher color purity in light-emitting elements.

The exemplary preparation steps of the light-emitting elements provided by the present application are shown in the following exemplary example 11.

### Example 11

In the light-emitting elements provided in this example, indium tin oxide (ITO) is used as the material for the anodes; HATCN is used as the material for the hole injection layers; TAPC is used as the material for the hole transport layers; TCTA is used as the material for the electron blocking layers; MCBP is used as the host material for the light-emitting layers of the light-emitting elements; organic compounds M1 to M10 in examples 1 to 10 and comparative compound 3 are used as guest materials for light-emitting layers of corresponding light-emitting elements, and Ir(ppy)₃ is used as the sensitizer for some light-emitting elements; POT2T is used as the material for the hole blocking layers; ANT-BIZ is used as the material for the electronic transport layers; Liq is used as the material for the electron injection layers; and Al is used as the material for the cathodes. In high vacuum conditions, the hole injection layer (with a thickness of 5 nm), the hole transport layer (with a thickness of 30 nm), the electron blocking layer (with a thickness of 15 nm), the light-emitting layer (with a thickness of 20 nm), the hole blocking layer (with a thickness of 10 nm), the electron transport layer (with a thickness of 40 nm), the electron injection layer (with a thickness of 1.5 nm), and the cathode (with a thickness of 100 nm) are sequentially deposited on a cleaned ITO substrate.

Specifically, in this example, light-emitting elements 1 to 20 and comparative elements 1 and 2 are prepared through the above steps. The organic compounds M1 to M10 are used as the guest materials in the light-emitting elements 1 to 10, respectively, and the comparative compound 3 is used as the guest material in the comparative element 1. The host material to the guest material in each of the light-emitting elements 1 to 10 and the comparative element 1 is 198:2 by mass. The organic compounds M1 to M10 are used as the guest materials in the light-emitting elements 11 to 20, respectively, and the comparative compound 3 is used as the guest material in the comparative element 2. The host material, to the sensitizer, and to the guest material in each of the light-emitting elements 11 to 20 and the comparative element 2 is 178:20:2 by mass.

Specifically, HATCN, TAPC, TCTA, mCBP, Ir(ppy)₃, POT2T, ANT-BIZ, Liq, and the comparative compound 3 (ref-1) can be commercially purchased. Structures of HATCN, TAPC, TCTA, mCBP, Ir(ppy)₃, POT2T, ANT-BIZ, Liq, and the comparative compound 3 are as follows:

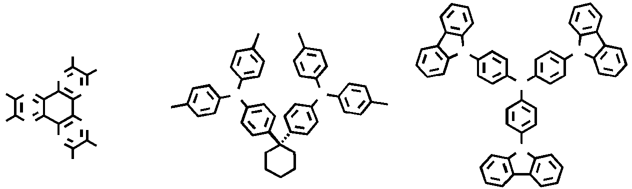

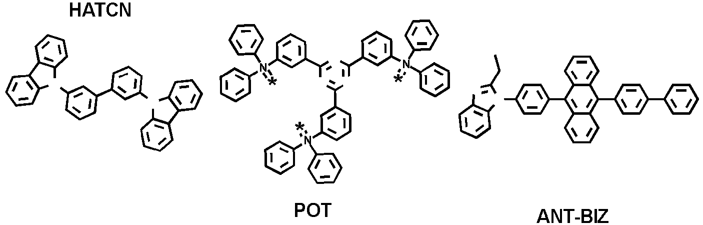

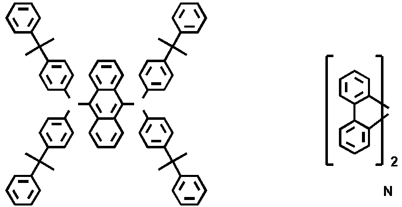

In this example, current-voltage (J-V) characteristics of the light-emitting elements 1 to 20 and the comparative elements 1 and 2 are tested at current density of 10 mA/cm², to obtain driving voltage (voltage (V)) at the same brightness, luminescence efficiency (CE (cd/A)), and time taken for brightness to decrease from initial brightness to 95% of initial brightness (LT95 (h)) of each light-emitting element and each comparative element. The results are shown in table 2.

**Table 2: Performance data of light-emitting elements 1 to 20 and comparative elements 1 and 2**

| | Voltage (V) | CE (cd/A) | LT95 (h) |
|---|---|---|---|
| Light-emitting element 1 | 3.5 | 59.6 | 60 |
| Light-emitting element 2 | 3.3 | 61.2 | 68 |
| Light-emitting element 3 | 3.4 | 66.3 | 74 |
| Light-emitting element 4 | 3.6 | 63.2 | 88 |
| Light-emitting element 5 | 3.4 | 67.3 | 95 |
| Light-emitting element 6 | 3.4 | 63.8 | 70 |
| Light-emitting element 7 | 3.4 | 66.4 | 74 |
| Light-emitting element 8 | 3.4 | 55.3 | 72 |
| Light-emitting element 9 | 3.4 | 66.2 | 77 |
| Light-emitting element 10 | 3.4 | 57.5 | 66 |
| Light-emitting element 11 | 3.2 | 108 | 70 |
| Light-emitting element 12 | 3.0 | 116 | 74 |
| Light-emitting element 13 | 3.2 | 128 | 78 |
| Light-emitting element 14 | 3.5 | 125 | 80 |
| Light-emitting element 15 | 3.3 | 136 | 85 |
| Light-emitting element 16 | 3.2 | 138 | 80 |
| Light-emitting element 17 | 3.2 | 123 | 85 |
| Light-emitting element 18 | 3.3 | 102 | 80 |
| Light-emitting element 19 | 3.2 | 128 | 82 |
| Light-emitting element 20 | 3.2 | 112 | 75 |
| Comparative element 1 | 3.6 | 13.8 | 70 |
| Comparative element 2 | 3.3 | 89 | 75 |

According to table 2, luminescence efficiency of light-emitting elements 1 to 10 obtained by using the guest materials M1 to M10 in the light-emitting layers are significantly improved compared with comparative element 1 under the same or equivalent driving voltage conditions; and luminescence efficiency of light-emitting elements 11 to 20 are significantly improved compared with comparative element 2 under the same or equivalent driving voltage conditions. Further, the light-emitting elements 1 to 10 have the luminescence efficiency ranging from 55.3 cd/A to 67.3 cd/A and the light-emitting elements 11 to 20 have the luminescence efficiency ranging from 102 cd/A to 138 cd/A, indicating that the addition of sensitizers effectively improves the luminescence efficiency of light-emitting elements. Moreover, compared with the time taken for the brightness to decrease from the initial brightness to 95% of the initial brightness of the comparative element 1, the time taken for the brightness to decrease from the same initial brightness to 95% of the initial brightness of the light-emitting elements 3 to 5 and 7 to 9 range from 72 hours to 95 hours, which are significant increased; and compared with the time taken for the brightness to decrease from the initial brightness to 95% of the initial brightness of the comparative element 2, the time taken for the brightness to decrease from the same initial brightness to 95% of the initial brightness of the light-emitting elements 13 to 19 range from 78 hours to 85 hours, which are significant increased. In addition, compared with the light-emitting elements 1 to 10, when using the same guest material, for the light-emitting elements 11 to 20, the time taken for the brightness to decrease from the same initial brightness to 95% of the initial brightness are further increased, indicating that the addition of sensitizers is conducive to further stabilizing the light-emitting elements and prolonging their life.

The light-emitting elements provided in the examples of the present application use the organic compounds with the multi-resonance effect. By elongating the distance between B and B atoms in the organic compound and/or introducing N atom(s) to break conjugation to weaken para-electronic coupling, the present application improves properties of the organic compound, thereby improving luminescence efficiency and color purity of display panels using the organic compound, and prolonging service life of display panels.

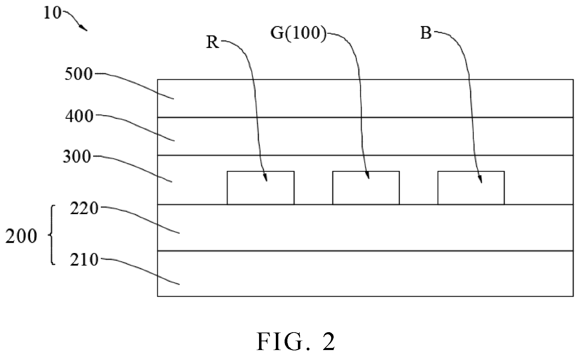

Referring to FIG. 2, embodiments of the present application further provide a display panel 10 that includes any light-emitting element 100 described above.

In some embodiments, the display panel 10 further includes an array substrate 200 disposed on a side of the light-emitting element 100.

The array substrate 200 includes a substrate 210, and the light-emitting element 100 is disposed on a side of the substrate 210.

In some embodiments, a first electrode layer 101 of the light-emitting element 100 is disposed on a side of the substrate 210, a second electrode layer 102 of the light-emitting element 100 is disposed on a side of the first electrode layer 101 away from the substrate 210, and an organic functional layer 103 is disposed between the first electrode layer 101 and the second electrode layer 102.

In some embodiments, the light-emitting element 100 is a green light-emitting element G, and the display panel 10 further includes a red light-emitting element R and a blue light-emitting element B.

In some embodiments, the array substrate 200 further includes a thin film transistor layer 220 disposed between the light-emitting element 100 and the substrate 210. The thin film transistor layer 220 includes a thin film transistor electrically connected to the first electrode layer 101 of the light-emitting element 100 to control luminescence of the light-emitting element 100.

In some embodiments, the display panel 10 further includes an encapsulation layer 300 disposed on a side of the light-emitting element 100 away from the array substrate 200 and covering the light-emitting element 100.

In some embodiments, the display panel 10 further includes a polarizing layer 400 disposed on a side of the encapsulation layer 300 away from the light-emitting element 100, and a cover layer 500 disposed on a side of the polarizing layer 400 away from the light-emitting element 100. The polarizing layer 400 can be replaced by a color film layer, which includes color resists and a black matrix disposed on two sides of each of the color resists.

The display panels provided in the embodiments of the present application use the organic compound with the multi-resonance effect. By elongating the distance between B and B atoms in the organic compound and/or introducing N atom(s) to break conjugation to weaken para-electronic coupling, the present application improves properties of the organic compound, thereby improving luminescence efficiency and color purity of the display panel using the organic compound, and prolonging service life of the display panel.

Embodiments of the present application provide the organic compound and the display panel. The organic compound has a structure represented by formula (1): 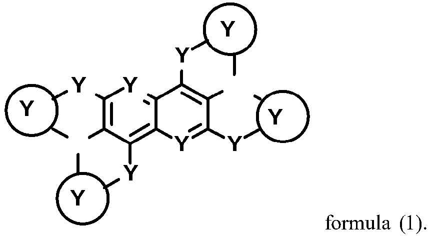 By providing the organic compound with the multi-resonance effect and elongating the distance between B and B atoms in the organic compound and/or introducing N atom(s) to break conjugation to weaken para-electronic coupling, the present application improves properties of the organic compound, thereby improving luminescence efficiency and color purity of the display panel using the organic compound, and prolonging service life of the display panel.

It can be understood that, for ordinary skilled in the art, equivalent replacements or changes can be made to the technical solutions based on the invention concepts of this disclosure, and all these changes or replacements fall within the scope of the claims attached to this description.

## Claims

1. An organic compound, having a structure represented by formula (1): 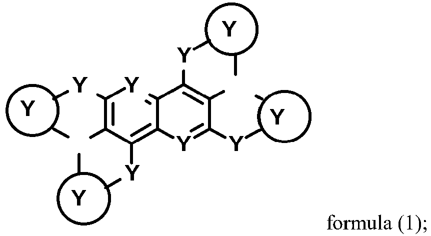
wherein M₁ and M₂ are each independently selected from B or N;
Y₁ and Y₂ are each independently selected from C or N, and at least one of Y₁ and Y₂ is N;
A₁, A₂, A₃, and A₄ are each independently selected from a substituted or unsubstituted aromatic group containing 6 to 30 carbon atoms or a substituted or unsubstituted heteroaromatic group containing 5 to 30 carbon atoms; A₁ and A₂ form a ring or do not form a ring together, and A₃ and A₄ form a ring or do not form a ring together;
when M₁ is B, X₁ and X₂ are each independently selected from electron-donating groups;
when M₁ is N, X₁ and X₂ are each independently selected from electron-withdrawing groups;
when M₂ is B, X₃ and X₄ are each independently selected from electron-donating groups; and
when M₂ is N, X₃ and X₄ are each independently selected from electron-withdrawing groups.

2. The organic compound of claim 1, wherein when M₁ is B, X₁ and X₂ are each independently selected from a group consisting of N-R₁, O, and S;
when M₁ is N, X₁ and X₂ are each independently selected from a group consisting of B-R₁, a carbonyl group, a sulfonyl group, and a sulfoxide group;
when M₂ is B, X₃ and X₄ are each independently selected from a group consisting of N-R₁, O, and S; and
when M₂ is N, X₃ and X₄ are each independently selected from a group consisting of B-R₁, a carbonyl group, a sulfonyl group, and a sulfoxide group; and
wherein R₁ is independently selected from H, D, T, a substituted or unsubstituted linear alkyl group containing 1 to 5 carbon atoms, a substituted or unsubstituted branched alkyl group containing 3 to 12 carbon atoms, a substituted or unsubstituted cycloalkyl group containing 3 to 12 carbon atoms, a substituted or unsubstituted aromatic group containing 6 to 15 carbon atoms, or a substituted or unsubstituted heteroaromatic group containing 5 to 15 carbon atoms at each occurrence.

3. The organic compound of claim 2, wherein at least one of A₁ to A₄ adjacent to R₁ is bridged with R₁ through a single bond or a first bridging group;
wherein the first bridging group is independently selected from a group consisting of N-R₂, CR₃R₄, SiR₅R₆, O, S, Se, Te, a carbonyl group, a sulfonyl group, and a sulfoxide group at each occurrence; and
R₂, R₃, R₄, R₅, and R₆ are each independently selected from H, D, T, a substituted or unsubstituted linear alkyl group containing 1 to 5 carbon atoms, a substituted or unsubstituted branched alkyl group containing 3 to 12 carbon atoms, a substituted or unsubstituted cycloalkyl group containing 3 to 12 carbon atoms, a substituted or unsubstituted aromatic group containing 6 to 15 carbon atoms, or a substituted or unsubstituted heteroaromatic group containing 5 to 15 carbon atoms at each occurrence.

4. The organic compound of claim 3, wherein the first bridging group is independently selected from a group consisting of 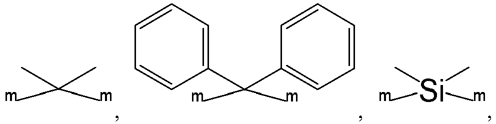 O, S, Se, Te, a carbonyl group, a sulfonyl group, and a sulfoxide group at each occurrence.

5. The organic compound of claim 1, wherein M₁ and M₂ are the same, and both Y₁ and Y₂ are N.

6. The organic compound of claim 5, wherein A₁, A₂, A₃, and A₄ are the same, and X₁, X₂, X₃, and X₄ are the same.

7. The organic compound of claim 6, wherein the organic compound has a structure represented by formula (1-1) or formula (1-2):

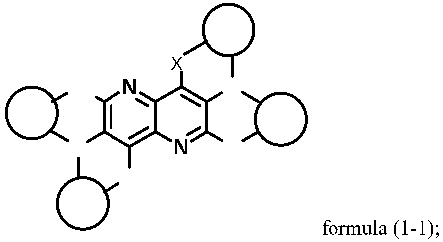

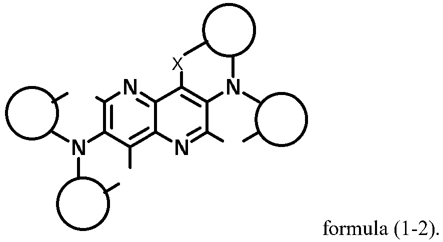

8. The organic compound of claim 1, wherein A₁, A₂, A₃, and A₄ are each independently selected from a substituted or unsubstituted aromatic group containing 6 to 15 carbon atoms or a substituted or unsubstituted heteroaromatic group containing 5 to 18 carbon atoms.

9. The organic compound of claim 1, wherein A₁ and A₂ are bridged through a single bond or a second bridging group, and A₃ and A₄ are bridged through a single bond or a third bridging group;
wherein the second bridging group and the third bridging group are each independently selected from a group consisting of a substituted or unsubstituted linear alkyl group containing 1 to 5 carbon atoms, a branched alkyl group, SiR₉R₁₀, O, S, Se, Te, a carbonyl group, a sulfonyl group, and a sulfoxide group at each occurrence; and
R₉ and R₁₀ are each independently selected from a substituted or unsubstituted linear alkyl group containing 1 to 5 carbon atoms, a branched alkyl group, or a substituted or unsubstituted aromatic group containing 6 to 10 carbon atoms at each occurrence.

10. The organic compound of claim 9, wherein both R₉ and R₁₀ are methyl.

11. The organic compound of claim 10, wherein the second bridging group and the third bridging group are each independently selected from a group consisting of 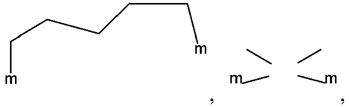 O, S, Se, Te, a carbonyl group, a sulfonyl group, and a sulfoxide group at each occurrence.

12. The organic compound of claim 1, wherein the organic compound is selected from a group consisting of the following compounds: 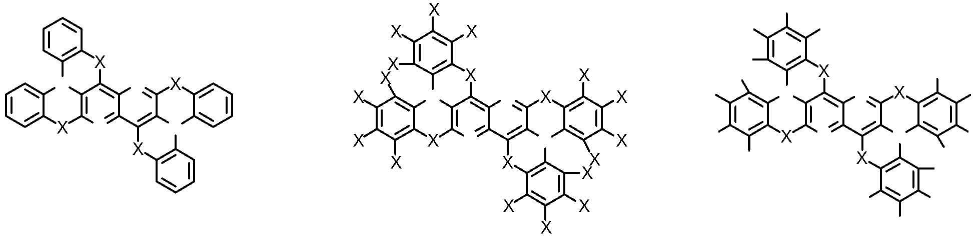 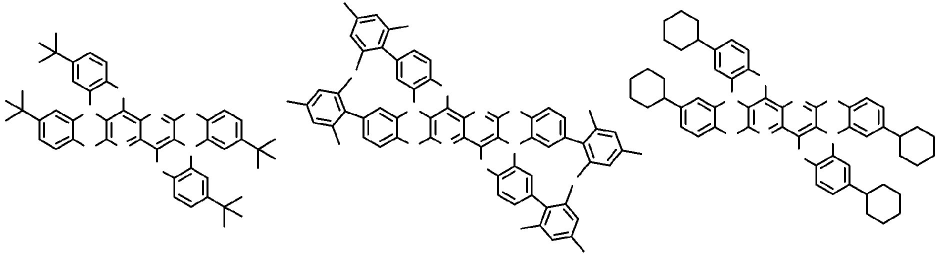 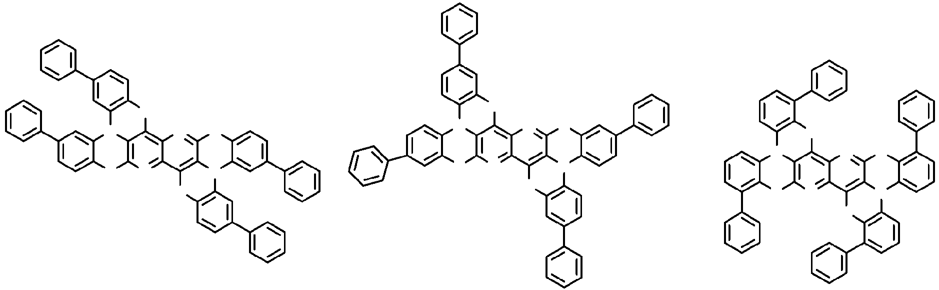 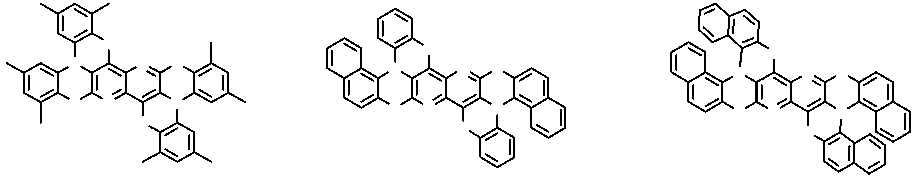 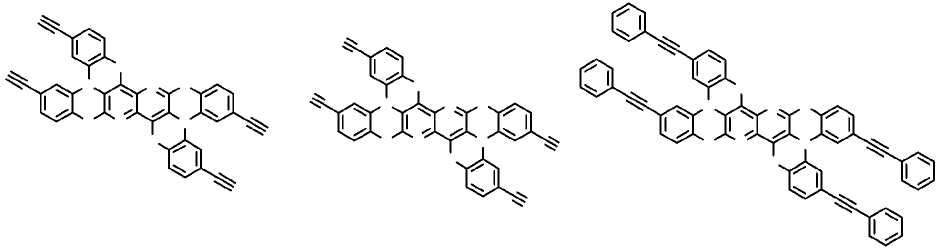 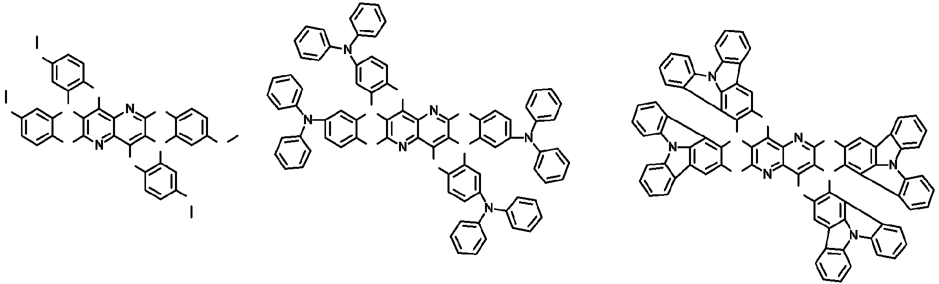 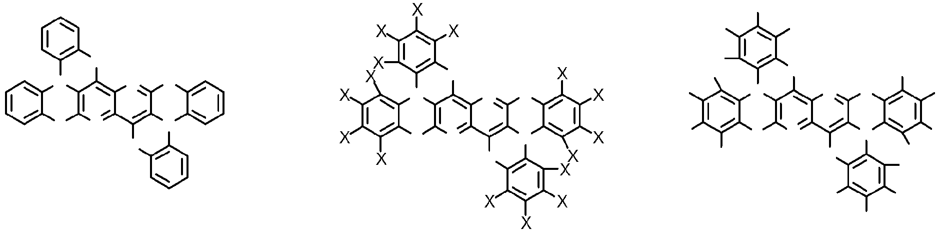 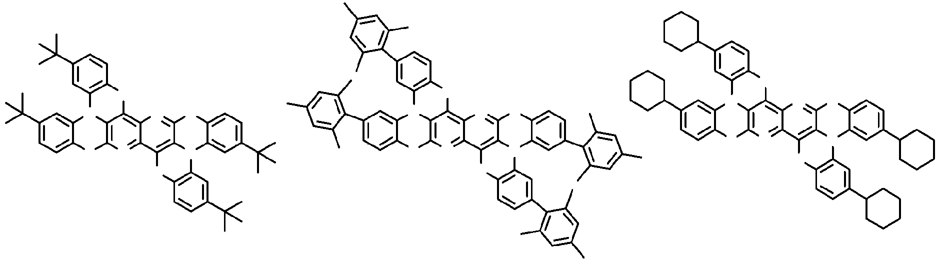 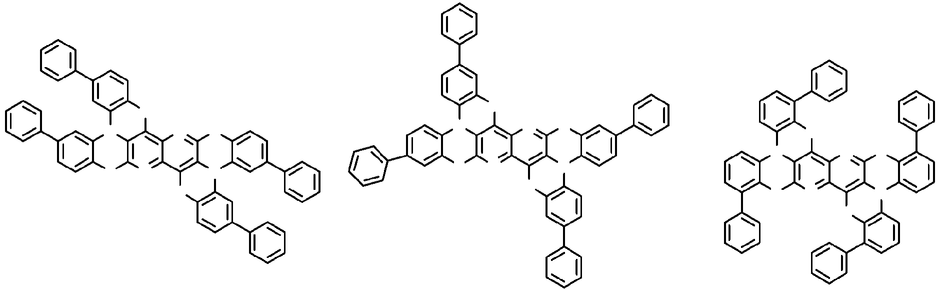 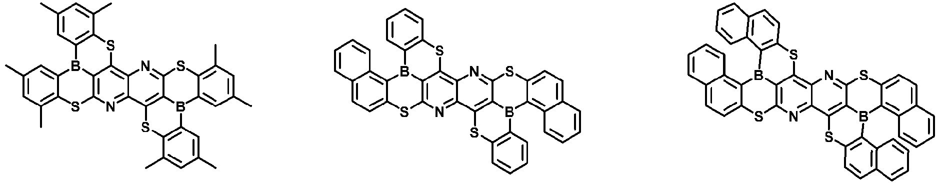 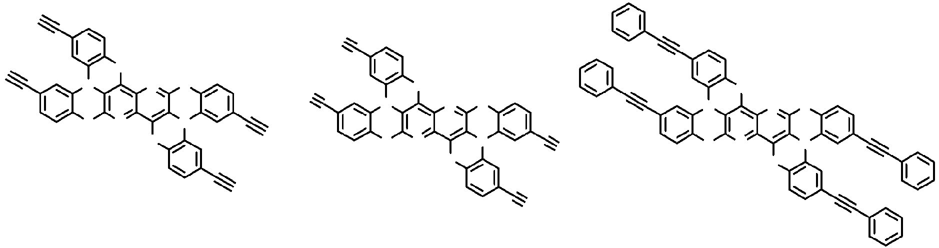 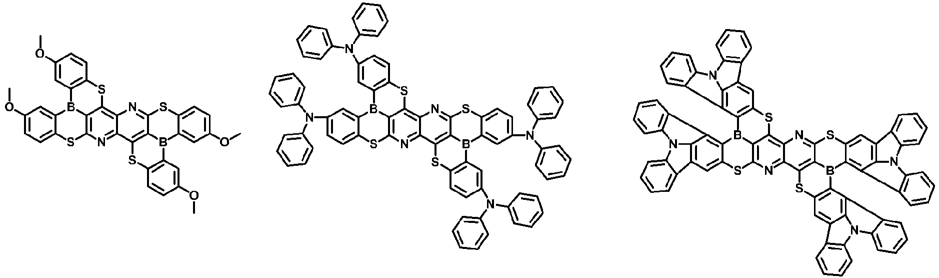 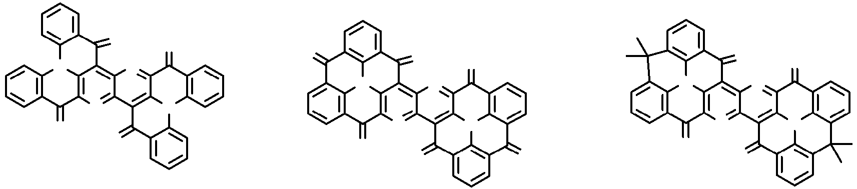 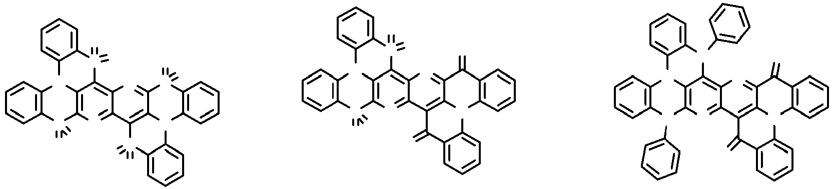 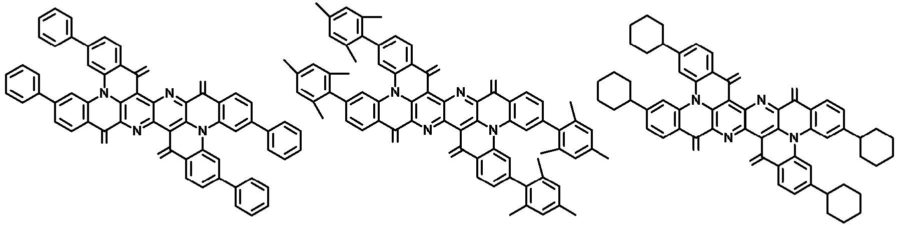 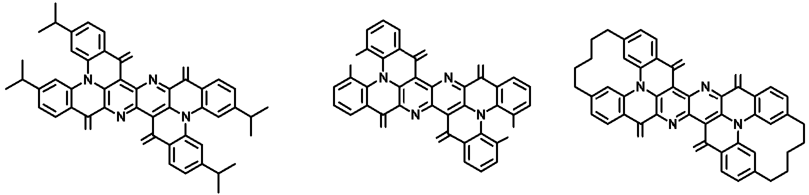 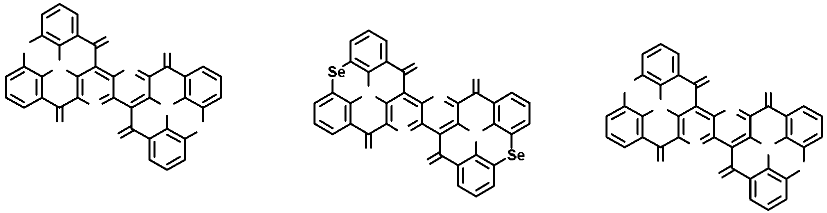 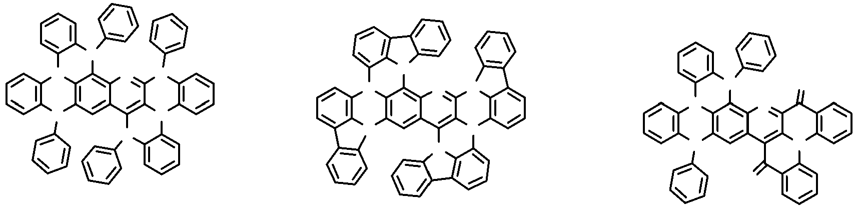 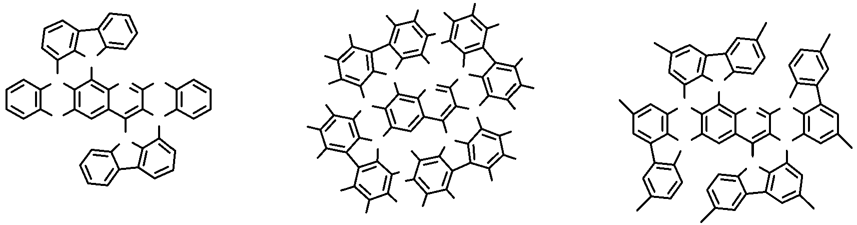 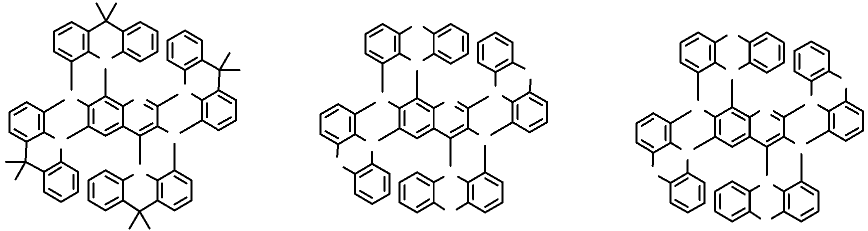 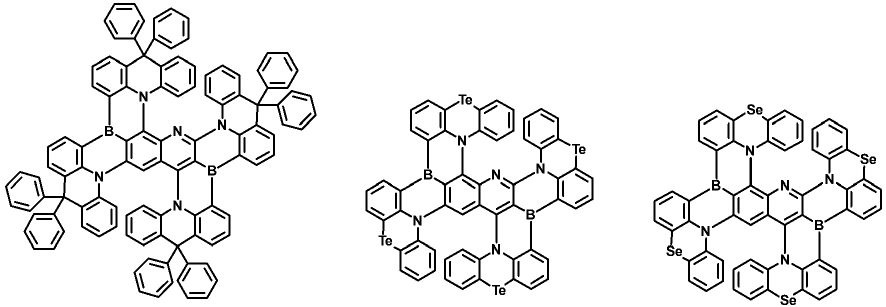 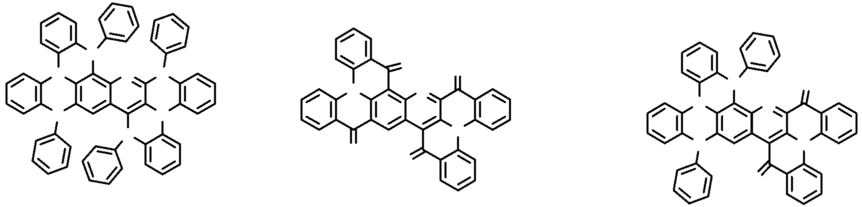 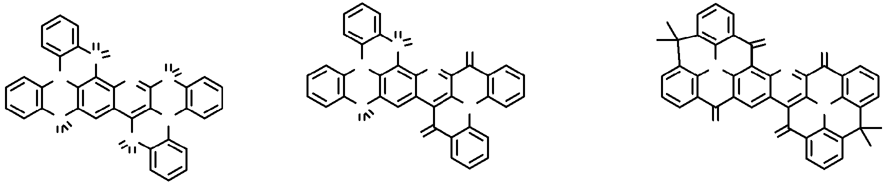 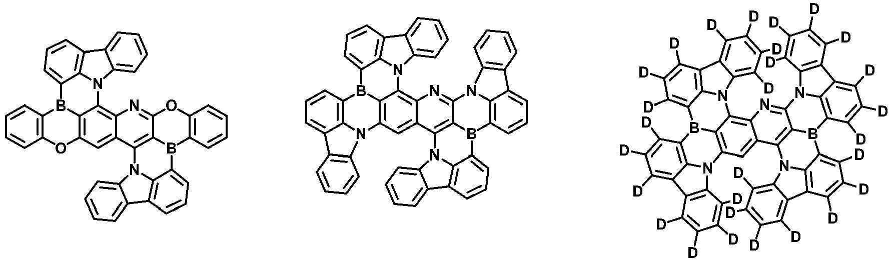 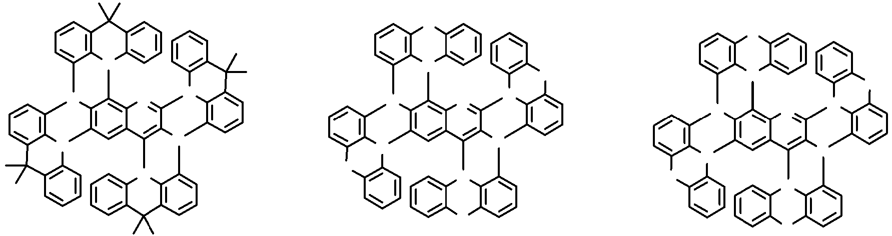 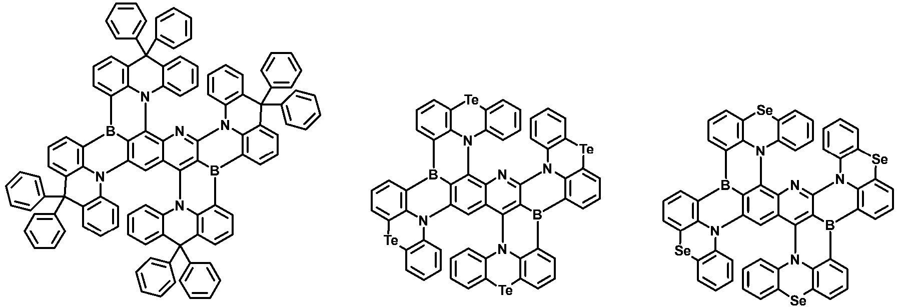 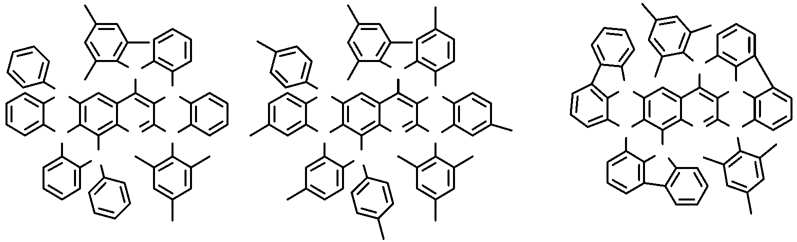 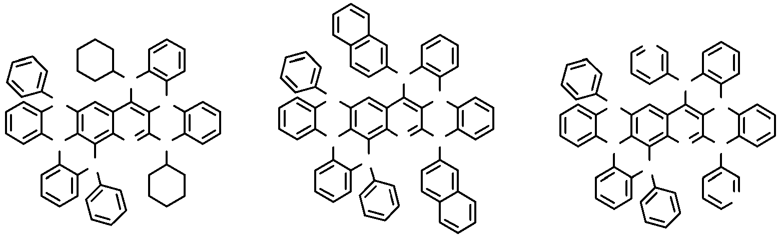 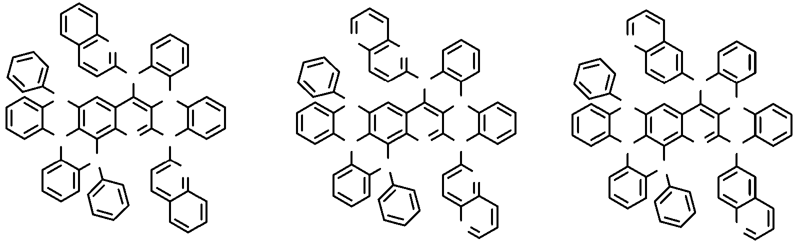 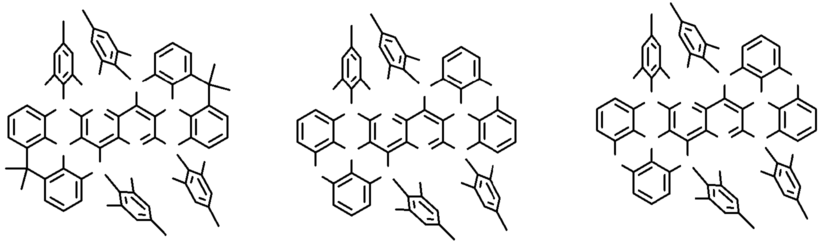 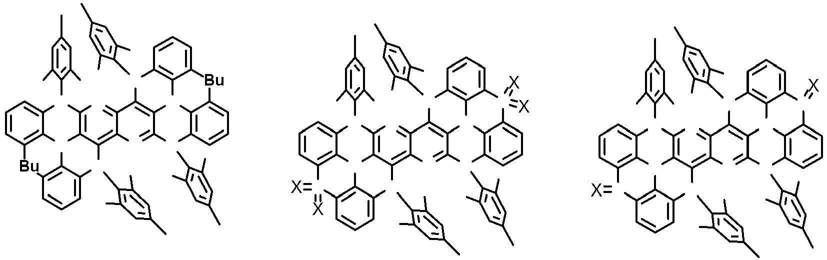 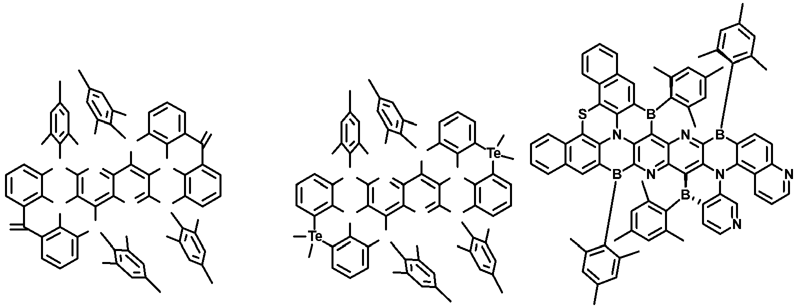 and

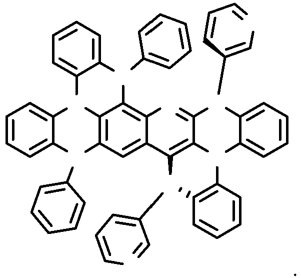

13. The organic compound of claim 1, wherein a highest occupied molecular orbital energy level of the organic compound is greater than or equal to -6.0 eV and greater than or equal to -5.0 eV; and
wherein a lowest unoccupied molecular orbital energy level of the organic compound is greater than or equal to -4.0 eV and greater than or equal to -3.0 eV.

14. A display panel, comprising:
a substrate;
a first electrode layer disposed on a side of the substrate;
a second electrode layer disposed on a side of the first electrode layer away from the substrate; and
an organic functional layer disposed between the first electrode layer and the second electrode layer;
wherein a material of the organic functional layer comprises an organic compound having a structure represented by formula (1): 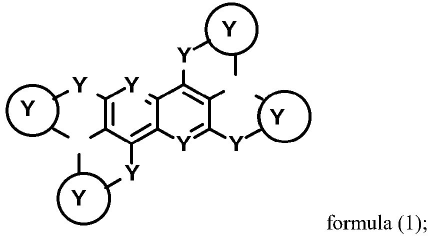
wherein M₁ and M₂ are each independently selected from B or N;
Y₁ and Y₂ are each independently selected from C or N, and at least one of Y₁ and Y₂ is N;
A₁, A₂, A₃, and A₄ are each independently selected from a substituted or unsubstituted aromatic group containing 6 to 30 carbon atoms or a substituted or unsubstituted heteroaromatic group containing 5 to 30 carbon atoms; A₁ and A₂ form a ring or do not form a ring together, and A₃ and A₄ form a ring or do not form a ring together;
when M₁ is B, X₁ and X₂ are each independently selected from electron-donating groups;
when M₁ is N, X₁ and X₂ are each independently selected from electron-withdrawing groups;
when M₂ is B, X₃ and X₄ are each independently selected from electron-donating groups; and
when M₂ is N, X₃ and X₄ are each independently selected from electron-withdrawing groups.

15. The display panel of claim 14, wherein the organic functional layer at least comprises a light-emitting layer comprising a host material and a guest material, wherein the guest material is one or more of the organic compounds.

16. The display panel of claim 14, wherein the organic functional layer comprises a light-emitting layer, wherein the light-emitting layer comprises a host material, a guest material, and a sensitizer, the guest material is one or more of the organic compounds, and the sensitizer is selected from at least one of a phosphorescence material and a thermally activated delayed fluorescence material.

17. The display panel of claim 14, wherein when M₁ is B, X₁ and X₂ are each independently selected from a group consisting of N-R₁, O, and S;
when M₁ is N, X₁ and X₂ are each independently selected from a group consisting of B-R₁, a carbonyl group, a sulfonyl group, and a sulfoxide group;
when M₂ is B, X₃ and X₄ are each independently selected from a group consisting of N-R₁, O, and S; and
when M₂ is N, X₃ and X₄ are each independently selected from a group consisting of B-R₁, a carbonyl group, a sulfonyl group, and a sulfoxide group;
wherein R₁ is independently selected from H, D, T, a substituted or unsubstituted linear alkyl group containing 1 to 5 carbon atoms, a substituted or unsubstituted branched alkyl group containing 3 to 12 carbon atoms, a substituted or unsubstituted cycloalkyl group containing 3 to 12 carbon atoms, a substituted or unsubstituted aromatic group containing 6 to 15 carbon atoms, or a substituted or unsubstituted heteroaromatic group containing 5 to 15 carbon atoms at each occurrence.

18. The display panel of claim 17, wherein at least one of A₁ to A₄ adjacent to R₁ is bridged with R₁ through a single bond or a first bridging group;
wherein the first bridging group is independently selected from a group consisting of N-R₂, CR₃R₄, SiR₅R₆, O, S, Se, Te, a carbonyl group, a sulfonyl group, and a sulfoxide group at each occurrence; and
R₂, R₃, R₄, R₅, and R₆ are each independently selected from H, D, T, a substituted or unsubstituted linear alkyl group containing 1 to 5 carbon atoms, a substituted or unsubstituted branched alkyl group containing 3 to 12 carbon atoms, a substituted or unsubstituted cycloalkyl group containing 3 to 12 carbon atoms, a substituted or unsubstituted aromatic group containing 6 to 15 carbon atoms, or a substituted or unsubstituted heteroaromatic group containing 5 to 15 carbon atoms at each occurrence.

19. The display panel of claim 14, wherein M₁ and M₂ are the same, and both Y₁ and Y₂ are N.

20. The display panel of claim 19, wherein A₁, A₂, A₃, and A₄ are the same, and X₁, X₂, X₃, and X₄ are the same.
